# EUROPEAN PATENT APPLICATION

(11) **EP 4 015 645 A1**
(43) Date of publication of application: **22.06.2022**
(21) Application number: 19941798.1
(22) Date of filing: 16.08.2019
(51) Int. Cl.: C12Q 1/68, G06K 9/00

(54) **BASE RECOGNITION METHOD AND SYSTEM, COMPUTER PROGRAM PRODUCT, AND SEQUENCING SYSTEM**

(71) Applicant: GeneMind Biosciences Company Limited, Luohu District, Shenzhen, Guangdong 518001 (CN)
(72) Inventor: LI, Linsen, Shenzhen, Guangdong 518001 (CN); JIN, Huan, Shenzhen, Guangdong 518001 (CN); XU, Weibin, Shenzhen, Guangdong 518001 (CN); JIANG, Zefei, Shenzhen, Guangdong 518001 (CN); SUN, Lei, Shenzhen, Guangdong 518001 (CN)
(74) Representative: Schiweck Weinzierl Koch Patentanwälte Partnerschaft mbB
(86) International application number: PCT/CN2019/101067
(87) International publication number: WO 2021/030952

(57) **Abstract**

Disclosed are a base recognition method and system, a computer program product, and a sequencing system. The base recognition method comprises: aligning to an image to be checked the coordinates of bright spots in a bright spot set of a corresponding sequencing template to determine corresponding coordinate positions on said image; determining the intensities of the corresponding coordinate positions on said image; and comparing the intensities of the corresponding coordinate positions on said image with a preset threshold, and performing base recognition on the basis of the information of the positions on said image having intensities greater than the preset threshold. In the method and/or system, by aligning to said image the coordinates of the bright spots in the bright spot set of the corresponding sequencing template, base recognition is performed on the basis of the information of the corresponding coordinate positions on said image, so that the bases can be recognized quickly, easily and with high throughput to finally determine the nucleic acid sequence.

## Description

### TECHNICAL FIELD

The present application relates to the field of information processing and identification, particularly to processing and analysis of data relevant to nucleic acid sequence determination, and more particularly to a method for base calling, a system for base calling, a sequencing system and a computer program product.

### BACKGROUND

In the related art, including in a sequencing platform performing multiple image acquisitions of a nucleic acid molecule (template) in biochemical reaction based on an imaging system to determine the nucleotide order of the nucleic acid molecule, how to effectively and accurately determine at least part of the nucleotide composition and order of a template nucleic acid in a high-throughput manner, as well as how to identify and process multiple images (including information in the images) acquired at different time points, is a matter of concern.

### SUMMARY

The embodiments of the present application are intended to solve at least one of the technical problems existing in the prior art or at least provide an alternative practical solution. To this end, the present application provides a method for base calling, a system for base calling, a computer program product and a sequencing system.

According to one embodiment of the present application, provided is a method for base calling, which comprises: aligning the coordinates of spots in a spot set corresponding to a sequencing template with an image to be detected so as to determine a corresponding coordinate position in the image to be detected, determining the intensity of the corresponding coordinate position in the image to be detected, comparing the intensity of the corresponding coordinate position in the image to be detected with a preset threshold, and performing base calling based on the information of the position in the image to be detected where the intensity is greater than the preset threshold, wherein the spot set of a corresponding sequencing template is acquired by construction based on a plurality of images; the images and the image to be detected are all acquired from base extensions and correspond to the same field of view; and a plurality of molecules carrying an optically detectable label are present in the field of view during the base extensions, and at least some of the nucleic acid molecules are present as spots in the images and/or the image to be detected.

According to one embodiment of the present application, provided is a system for base calling for implementing the method for base calling according to any of the aforementioned embodiments of the present application, the system comprising: a aligning module for aligning the coordinates of spots in a spot set corresponding to a sequencing template with an image to be detected so as to determine a corresponding coordinate position in the image to be detected, an intensity determination module for calculating the intensity of the corresponding coordinate position in the image to be detected from the aligning module, and a calling module for comparing the intensity of the corresponding coordinate position in the image to be detected from the intensity determination module with a preset threshold and performing base calling based on the information of the position in the image to be detected where the intensity is greater than the preset threshold, wherein the spot set of a corresponding sequencing template is acquired by construction based on a plurality of images; the images and the image to be detected are all acquired from base extensions and correspond to the same field of view; and a plurality of molecules carrying an optically detectable label are present in the field of view during the base extensions, and at least some of the nucleic acid molecules are present as spots in the images and/or the image to be detected.

According to another embodiment of the present application, provided is a system for base calling, which comprises: a memory for storing data including a computer-executable program, a processor for executing the computer-executable program to implement the method for base calling according to any of the aforementioned embodiments of the present application.

According to one embodiment of the present application, provided is a sequencing system, which comprises the system for base calling according to any of the aforementioned embodiments of the present application.

According to one embodiment of the present application, provided is a computer-readable storage medium for storing a program executable by a computer, wherein executing the program comprises implementing the method for base calling according to any of the aforementioned embodiments. The computer-readable storage medium includes, but is not limited to, read-only memories, random access memories, magnetic disks, optical disks, or the like. According to one embodiment of the present application, provided is a computer program product comprising an instruction, wherein the instruction causes a computer to execute the method for base calling according to any of the aforementioned embodiments of the present application when the program is executed by a computer.

According to one embodiment of the present application, provided is a sequencing system comprising the computer program product according to any of the aforementioned embodiments of the present application.

With the method for base calling, the system for base calling, the computer-readable storage medium, the computer program product and/or the sequencing system according to any of the aforementioned embodiments, by aligning the coordinates of spots in a spot set corresponding to a sequencing template with an image to be detected and performing base calling based on the information of a corresponding coordinate position in the image to be detected, the types and sequence of bases bound to the template nucleic acid during base extension can be simply and efficiently identified in a high-throughput manner, and the nucleic acid sequence of the template can be simply, quickly and accurately determined.

The additional aspects and advantages of the embodiments of the present application will be partially set forth in the following description, and will partially become apparent from the following description or be appreciated by practice of the embodiments of the present application.

### BRIEF DESCRIPTION OF THE DRAWINGS

FIG. 1 is a schematic flowchart of a method for base calling according to a specific embodiment of the present application.
FIG. 2 is a schematic flowchart of a method for base calling according to a specific embodiment of the present application.
FIG. 3 is a schematic of a process and a result of merging the spots in images Repeat1-20 to obtain a spot set corresponding to a sequencing template according to a specific embodiment of the present application.
FIG. 4 is a schematic of a correction process and a correction result according to a specific embodiment of the present application.
FIG. 5 is a schematic of a matrix corresponding to a candidate spot and pixel connectivity according to a specific embodiment of the present application.
FIG. 6 is a schematic of pixel values in an m1 × m2 area centered on a center pixel of a pixel matrix according to a specific embodiment of the present application.
FIG. 7 is a schematic showing the comparison between spot detection results obtained before and after determination based on a second spot detection threshold according to a specific embodiment of the present application.
FIG. 8 shows a histogram of pixel values distribution of pixels in a particular 300 × 300 block according to the according to a specific embodiment of the present application.
FIG. 9 shows a histogram of pixel values in a particular 300 × 300 block according to a specific embodiment of the present application.
FIG. 10 is a structural schematic of a system for base calling according to a specific embodiment of the present application.

### DETAILED DESCRIPTION

The embodiments of the present disclosure are described in detail below, and the examples of the embodiments are shown in the accompanying drawings, throughout which identical or similar reference numerals represent identical or similar elements or elements having identical or similar functions. The embodiments described below by reference to the drawings are exemplary and are merely intended to explain the present application rather than be construed as limiting the present application.

In the description herein, the terms "first", "second", "third" and "fourth" are used for description purpose only rather than be construed as indicating or implying relative importance or implicitly indicating the number or sequence of indicated technical features. In the description of the present application, unless otherwise specifically defined, "a plurality of' means two or more.

In the description herein, the term "spot" or "peak" refers to luminous spots or points in an image, and a luminous spot occupies at least one pixel.

In the description herein, the term "sequencing", also referred to as sequence determination or gene sequencing, refers to nucleic acid sequence determination, including DNA sequencing and/or RNA sequencing, including long fragment sequencing and/or short fragment sequencing, and including identification of the types and the determination of the sequence of bases in a plurality of continuous or non-continuous specific positions of a nucleic acid sequence; sequencing by synthesis (SBS) or sequencing by ligation (SBL) may be used, including a process in which a nucleotide or nucleotide analogue is bound to a template, i.e., base extension. Detection of the "spot" refers to the detection of an optical signal from an extended base or base cluster.

Sequencing can be performed through a sequencing platform, which may be selected from, but is not limited to, the Hiseq/Miseq/Nextseq/Novaseq sequencing platform (Illumina), the Ion Torrent platform (Thermo Fisher/Life Technologies), the BGISEQ and MGISEQ platforms (BGI) and single-molecule sequencing platforms. The sequencing method may be selected from single-read sequencing and paired-end sequencing. The obtained sequencing results/data (i.e., read fragments) are referred to as reads. The length of a read is referred to as read length.

In the description herein, images acquired from sequencing/base extension or images obtained by conversion or construction based on these images may be grayscale images or color images. For a grayscale image, the pixel value refers to the grayscale value; for a 16-bit grayscale image such as tiff grayscale image, the pixel value ranges from 0 to 65535; for an 8-bit grayscale image, the pixel value ranges from 0 to 255. For a color image, each pixel has three pixel values. Image detection/target information identification can be perform directly based on an array of the pixel values using the provided method and/or system; alternatively, the color image can be converted into a grayscale image first, which is then processed and subjected to information identification, thereby reducing the calculation and complexity in the image detection and signal identification processes. A non-grayscale image may be converted into a grayscale image with methods including but not limited to floating point algorithm, integer method, shift method, mean value method, etc.

In the description herein, unless otherwise specifically defined, based on image information, the terms "intensity" and pixel (pixel value) are used interchangeably, and the intensity or pixel value may be a real or objective absolute value, or may be a relative value including various variations based on the real pixel value, such as an increased pixel value, a reduced pixel value, a proportion or relationship based on the pixel value. Generally, when comparison between a plurality of images or spots or positions in intensity/pixel value is involved, the intensity/pixel value of the images or spots or positions is the intensity/pixel size after the same processing, such as objective pixel values or pixel values after the same transformation; and when comparison and analysis based on information of particular positions in one or more images are involved and the particular positions are determined, the images are preferably aligned and kept in the same coordinate system when determining these particular positions.

In a platform for determining a nucleic acid sequence based on optical imaging, especially a single-molecule sequencing platform, as the signal from the target molecule is weak and the signal intensity will alter in a short time, after an image is acquired, for base calling, generally, spots in the acquired image will be first detected and identified, i.e., real signals from extended bases or base clusters will be detected and identified, and then each of the spots will be matched with a spot on a sequencing template. For example, the spots in the image to be detected are traversed, and if a certain spot in the image to be detected and a spot on the sequencing template are close enough (which is related to the resolution and the like), the two spots are considered to overlap, a target nucleic acid molecule is considered to exist in the position corresponding to the spot in the image to be detected, and the target nucleic acid molecule is involved in nucleotide binding reaction (base extension), thereby identifying the type of the nucleotide/base bound to the target nucleic acid molecule. In this commonly used method for base calling, whether the nucleic acid molecule represented on the sequencing template reacts (emits light; base extension) in the current cycle is determined by comparing the distance between the coordinates of the spot in the image to be detected and the coordinates of the spot on the sequencing template; if it does react, the type of the base added is read or the type of the base corresponding to the nucleic acid molecule is read. After testing based on a large amount of data, the inventor found that this method is seriously influenced by image quality, spot positioning algorithm, spot density distribution and the like, and base are likely to be called mistakenly.

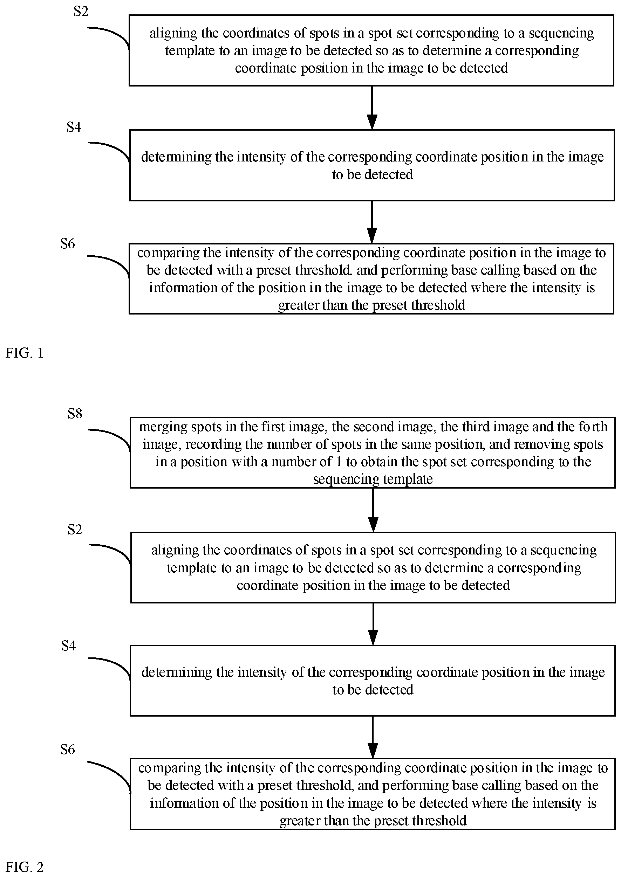

Thus, as shown in FIG. 1, the inventor provides a method for base calling in one embodiment, the method comprising: S2, aligning the coordinates of spots in a spot set corresponding to a sequencing template with an image to be detected so as to determine a corresponding coordinate position in the image to be detected; S4, determining the intensity of the corresponding coordinate position in the image to be detected; and S6, comparing the intensity of the corresponding coordinate position in the image to be detected with a preset threshold, and performing base calling based on the information of the position in the image to be detected where the intensity is greater than the preset threshold. The spot set corresponding to the sequencing template is acquired by construction based on a plurality of images; the images and the image to be detected are all acquired from base extensions and from the same field of view; and a plurality of molecules carrying an optically detectable label are present in the field of view during the base extensions, and at least some of the nucleic acid molecules are present as spots in the images and/or the image to be detected. The nucleic acid molecule is a template to be sequenced or a nucleic acid complex comprising the template, and the base extension comprises a process in which nucleotides, including nucleotide analogues, are bound to the template or nucleic acid complex; it is known to those skilled in the art that in an image obtained on a sequencing platform for determining sequences based on optical detection, nucleotides or nucleic acid molecules carrying an optically detectable label such as a fluorescent molecule, are excited by laser to emit light, and thus appear in the image as spots, each of which occupies several pixels.

In the method for base calling, the coordinates of spots in a spot set corresponding to a sequencing template are aligned with an image to be detected and base calling is performed based on the information of a corresponding coordinate position in the image to be detected. Compared to a general method in which spots in an image to be detected is detected and base calling is performed by determining whether the detected spots can match specific spots in a spot set corresponding to a sequencing template, this method can be used to simply and efficiently identify the types and sequence of bases bound to the template nucleic acid during base extensions in a high-throughput manner, so that the sequence of the template nucleic acid can be simply, quickly and accurately determined. Comparative testing further reveals that the amount of reads obtained by this method, including reads corresponding to unique positions of a reference sequence, is 30% or more greater than that obtained by a general method for base calling. The spot set corresponding to the sequencing template can be constructed during the implementation of the method for base calling or may be constructed in advance. In one example, the spot set corresponding to the sequencing template is constructed based on images in advance and saved for later use. Specifically, as shown in FIG. 2, the spot set corresponding to the sequencing template is constructed based on images. The images include a first image, a second image, a third image and a fourth image corresponding to the same field of view during base extensions of four types of bases, namely base/nucleotide/nucleotide analogues A, T/U, G and C, respectively. The first image comprises an image M1 and an image M2, the second image comprises an image N1 and an image N2, the third image comprises an image P1 and an image P2, and the fourth image comprises an image Q1 and an image Q2. Sequential and/or simultaneous completion of base extensions of the four types of bases once is defined as one cycle of sequencing. The images M1 and M2 separately come from two cycles of sequencing, the images N1 and N2 separately come from two cycles of sequencing, the images P1 and P2 separately come from two cycles of sequencing, and the images Q1 and Q2 separately come from two cycles of sequencing. The method comprises: S8, merging spots in the first image, the second image, the third image and the forth image, recording the number of spots in the same position, and removing spots with a number of 1 to obtain the spot set corresponding to the sequencing template. In this method, spots in the images are directly merged, so that the spot set corresponding to the nucleic acid molecule (sequencing template) can be quickly and easily constructed. The constructed spot set can effectively, accurately and comprehensively reflect the information of the sequencing template and is favorable for subsequent accurate base calling, thereby obtaining an accurate nucleic acid sequence.

The one cycle of sequencing, i.e., sequential and/or simultaneous completion of base extensions of four types of bases once, may be one characterized by simultaneous extensions of four types of base substrates (such as nucleotide analogues/base analogues) in one base extension system, one characterized by extensions of two types of base analogues in one base extension system and extensions of the other two types of the substrates in the next base extension system, or one characterized by separate extensions of four types of base analogues in four sequential base extension systems. It can be known that the first image, the second image, the third image and the fourth image may be acquired from two or more base extensions. In addition, a base extension may involve one or more image acquisitions.

In one example, one cycle of sequencing comprises multiple base extensions, such as one-color sequencing, in which the substrates (nucleotide analogues) corresponding to four types of bases contain the same fluorescent dye. One cycle of sequencing comprises four base extensions (4 repeats). For a field of view, one base extension involves one image acquisition, with the images M1, N1, P1 and Q1 separately acquired from the same field of view of the four base extensions in one cycle of sequencing.

In another example, one cycle of sequencing comprises two base extensions, such as a two-color sequencing, in which two of the substrates (nucleotide analogues) corresponding to four types of bases used contain one fluorescent dye and the other two substrates contain another fluorescent dye with a different excitation wavelength. One cycle of sequencing comprises two base extensions, and the two types of base substrates with different dyes are subjected to a nucleotide binding/extension in one base extension. For a field of view, one base extension involves two image acquisitions at different excitation wavelengths, with the images M1, N1, P1 and Q1 separately acquired from the same field of view at two excitation wavelengths in two base extensions of one cycle of sequencing.

In yet another example, one cycle of sequencing comprises one base extension, such as a two-color sequencing on a next generation sequencing platform, in which four types of base substrates (such as nucleotide analogues) contain dye a, dye b, dye a and dye b, and no dye, respectively, with the dye a and the dye b having different excitation wavelengths. The four types of the substrates enable one cycle of sequencing in the same base extension. One base extension involves two image acquisitions at different excitation wavelengths, with the first image and the third image, the second image and the fourth image, and the image M1 and the image N1 separately acquired from the same field of view of different cycles of sequencing or the same field of view at different excitation wavelengths of the same cycle of sequencing.

In yet another example, one cycle of sequencing comprises one base extension, such as a four-color sequencing on a next generation sequencing platform, in which four types of base substrates (such as nucleotide analogues) contain dye a, dye b, dye c and dye d, respectively, with the dye a, dye b, dye c and dye d having different excitation wavelengths. The four types of the substrates enable one cycle of sequencing in the same base extension. One base extension involves four image acquisitions at different excitation wavelengths, with the image M1, the image N1, the image P1 and the image Q1 separately acquired from the same field of view of different cycles of sequencing or the same field of view at different excitation wavelengths of the same cycle of sequencing.

When designing an algorithm for constructing a sequencing template, the inventor found that in some template construction algorithms, as some spots are discarded in the template construction process, and generally, the image information acquired from a field of view of the first cycle of sequencing has far more influence than the image acquired from the field of view of subsequent sequencing on the template construction, spots corresponding to the sequencing template are easily lost. In some specific embodiments, S8 comprises: (a) merging spots in the image N1 in the image M1 to obtain a one-time merging image M1, counting and marking overlap spots in the one-time merging image M1 according to the number of spots contained in the overlap spots, and marking non-overlap spots as 1, wherein a plurality of spots distanced by less than a first predetermined pixel in the one-time merging image M1 are counted as one overlap spot; (b) replacing the image N1 with the image PI, the image Q1, the image M2, the image N2, the image P2 or the image Q2, replacing the image M1 with the one-time merging image M1, and repeating (a) multiple times until the spots in all of the images are merged to obtain an original spot set; and (c) removing spots marked as 1 in the original spot set to obtain the spot set corresponding to the sequencing template. As such, the weights of the images from different cycles of sequencing can be balanced, so that more accurate template spots can be obtained, and the spot set corresponding to the sequencing template can be quickly and easily obtained, which is favorable for accurate base calling to obtain reads.

In one example, in the imaging system used, the size of an electronic sensor is 6.5 µm, the magnification of a microscope is 60×, and the smallest size that can be seen is 0.1 µm. The size of a spot corresponding to a nucleic acid molecule is generally less than 10 × 10 pixels. In one example, the first predetermined pixel is set to be 1.05 pixel. As such, overlap spots can be accurately determined, and a sequencing template (spot set) can be accurately constructed.

In one example, a TemplateVec is provided to carry the merging result of spots (peaks) in the images Repeats 1-20 from the same field of view; the successfully merged spots are counted in each merging; after all mergings are completed, the spots with a count of 1 are removed. Specifically, when peaks in the image Repeat1 are merged into TemplateVec, since no spots are initially present in TemplateVec, the total number of spots in TemplateVec equals the number of spots in the image Repeat1, and the count for each of the spots is 1; when spots in the image Repeat2 are merged into TemplateVec, distances between the spots in Repeat2 and the spots in TemplateVec are determined, if a distance less than 1.05 pixels occurs, the corresponding two spots will be merged to obtain an overlap spot, wherein the position of any of the two spots or the average position of the two spots is taken as the position of the overlap spot, and the count of the overlap spot is increased by 1; if no distance less than 1.05 pixels occurs, the spots will be appended to TemplateVec, with the count being 1. The above procedures are repeated until the spots in the image Repeat20 are merged into TemplateVec. Finally, the spots in TemplateVec are screened to remove spots with a count of 1.

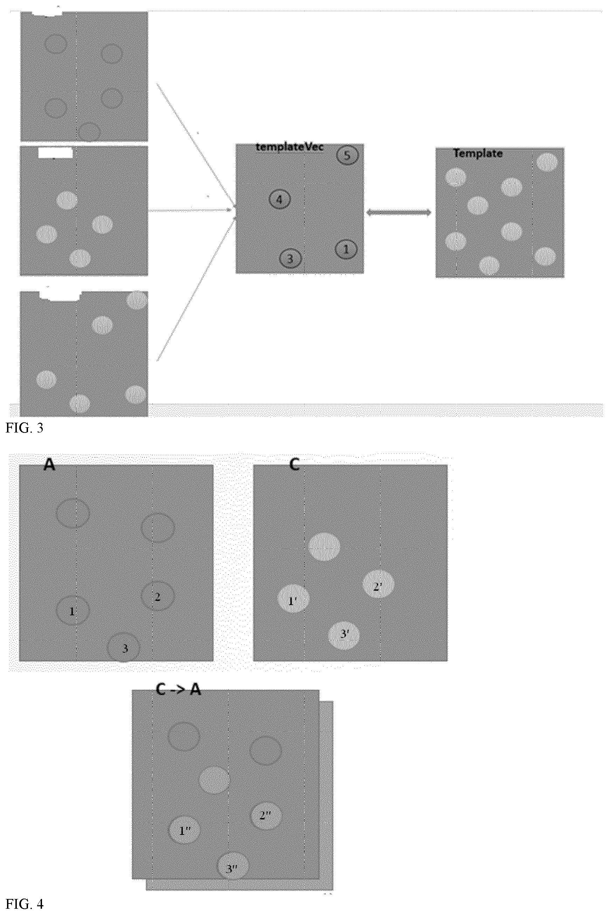

Due to different sequences of addition, some early added spots that are far apart from one another will become close to one another. In one example, spots that are less than 1.05 pixels apart in TemplateVec are further merged again. As such, a more accurate sequencing template can be obtained. FIG. 3 shows the above sequencing template construction process, and the circles in the figure indicates the spots.

For the case where multiple spots are merged into one spot, the position/coordinates of the merged spot can be determined, in some examples, according to the barycentric coordinates of a plurality of spots before merging. For example, the barycentric coordinates of any of the plurality of spots or average barycentric coordinates of the plurality of spots are taken as the coordinates of the merged spot. For another example, weights are set according to the coordinates of the plurality of spots so as to determine the coordinates of the merged spot; for example, different contribution values are set according to the number of spots comprised in one spot before merging and/or the sequencing cycle each of the spots is from before merging, that is, different weights are set for the coordinates of the spots so as to determine the coordinates of the merged spot. As such, spot information that accurately reflects the reality (corresponding sequencing template) can be obtained. In one example, a higher weight is set for a spot that comprises a plurality of spots before merging, and/or a higher weight is set for a spot that comes from the image of the first cycle of sequencing before merging. As such, the information of the merged spot can objectively and accurately reflect the information before the merging, an accurate spot set corresponding to the sequencing template can be constructed, and bases can be accurately called. In some specific embodiments, the images are registered images. As such, a spot set corresponding to the sequencing template can be acquired accurately, and bases can be accurately called.

The method for image registration (i.e., image correction) is not limited. In some examples, the following method is employed to perform image registration, comprising: performing a first registration for an image to be registered based on a reference image, wherein the reference image and the image to be registered correspond to the same object and each comprise a plurality of spots, comprising: determining a first offset between a predetermined block in the image to be registered and a corresponding predetermined block in the reference image, and moving all spots in the image to be registered based on the first offset to obtain an image to be registered having undergone the first registration; and performing a second registration for the image to be registered having undergone the first registration based on the reference image, comprising: merging the image to be registered having undergone the first registration with the reference image to obtain a merging image, calculating an offset of all overlap spots in a predetermined block in the merging image to determine a second offset, two or more spots distanced by less than a predetermined pixel being defined as an overlap spot, and moving all spots in the image to be registered having undergone the first registration based on the second offset to register the image to be registered. The method for image registration through two associated registrations, which can be relatively referred to as coarse registration and fine registration including fine registration using spots in an image, can quickly complete high-precision image correction based on a small quantity of data, and is particularly suitable for scenarios where high-precision image correction is required, e.g., detection of images at the single molecule level, such as images acquired from sequencing on a third generation sequencing platform. The single molecule level means that resolution is the size of a single molecule or a few molecules, such as no more than 10, 8, 5, 4 or 3 molecules.

In some specific embodiments, the "spot" corresponds to the optical signal of an extended base or base cluster or the interference signal of other luminescent substances. The predetermined block in an image may be all or part of the image. In one example, the predetermined block in an image is part of the image, e.g., a 512 × 512 block at a center of the image. The center of the image is a center of the field of view. The point of intersection between the optical axis of the imaging system and the imaging plane may be referred to as a central point of the image, and a block centered on the central point may be regarded as a central block of the image.

In some specific embodiments, the image to be registered is acquired from a nucleic acid sequencing platform.

Specifically, the image to be registered is acquired from a nucleic acid sequencing platform that performs sequencing based on optical imaging, the platform comprising an imaging system and a nucleic acid sample carrier system. The target nucleic acid molecules with an optically detectable label are immobilized in a reactor, which is also referred to as a flowcell. The flowcell is fixed on a movable carrier, and is driven by the movable carrier to acquire images of the target nucleic acid molecules at different positions (different fields of view) in the flowcell. In general, precision limitations exist in the movement of the optical system and/or the movable carrier. For example, there may be a deviation between an arrival position specified by an instruction and a position at which the mechanical structure actually arrives, particularly in application scenarios requiring high precision. Therefore, in the process of moving hardware according to an instruction to perform multiple image acquisitions on the same position (field of view) at different time points, it is difficult to completely align the images of the same field of view acquired at the different time points. Correcting and aligning these images can help to accurately determine the nucleotide sequence of a nucleic acid molecule according to the change of information in the images acquired at multiple time points.

In some specific embodiments, the reference image is acquired by construction, and can be constructed when registration is performed for the image to be registered, or can be constructed in advance for calling when needed.

In some examples, constructing a reference image comprises: acquiring a fifth image and a sixth image, wherein the fifth image and the sixth image correspond to the same field of view/object as the image to be registered; performing a coarse registration for the sixth image based on the fifth image, comprising: determining an offset between the sixth image and the fifth image, and moving the sixth image based on the offset to obtain a sixth image having undergone the coarse registration; and merging the fifth image with the sixth image having undergone the coarse registration to obtain the reference image, wherein the fifth image and the sixth image each comprise a plurality of spots. As such, a reference image containing more or relatively more complete information is acquired by construction and used as a basis for correction, which can facilitate more accurate image registration. For images acquired by nucleic acid sequence determination, constructing a reference image using multiple images helps to provide complete spot information corresponding to the nucleic acid molecule using the reference image, and is favorable for image correction based on spots and further for the acquisition of a spot set corresponding to the sequencing template and base calling.

In some embodiments, the fifth image and the sixth image are acquired at different times from the same field of view of a nucleic acid sequence determination (sequencing). In one example, one cycle of sequencing comprises a plurality of base extensions, such as one-color sequencing in which substrates (nucleotide analogues) corresponding to four types of bases used contain the same fluorescent dye. One cycle of sequencing comprises four base extensions (4 repeats). For a field of view, one base extension involves one image acquisition, with the fifth image and the sixth image acquired from the same field of view of different base extensions. As such, the reference image obtained by processing and collecting information from the fifth image and the sixth image is used as a basis for correction, which can facilitate more accurate image correction.

In another example, single-molecule two-color sequencing is performed, wherein two of the substrates (nucleotide analogues) corresponding to four types of bases used contain one fluorescent dye and the other two substrates contain another fluorescent dye with a different excitation wavelength and an emission wavelength. One cycle of sequencing comprises two base extensions, and the two types of base substrates with different dyes are subjected to a binding reaction in a base extension. For a field of view, one base extension involves two image acquisitions at different excitation wavelengths, with the fifth image and the sixth image separately acquired from the same field of view of different base extensions or the same field of view at different excitation wavelengths of the same base extension. As such, the reference image obtained by processing and collecting information from the fifth image and the sixth image is used as a basis for correction, which can facilitate more accurate image correction.

In yet another example, one cycle of sequencing comprises one base extension, such as a two-color sequencing on a second generation sequencing platform, in which four types of base substrates (such as nucleotide analogues) contain dye a, dye b, dye a and dye b, and no dye, respectively, with the dye a and the dye b having different emission wavelengths when excited; or such as a four-color sequencing, in which four types of base substrates (such as nucleotide analogues) contain dye a, dye b, dye c and dye d, respectively, with the dye a, dye b, dye c and dye d having different emission wavelengths when excited. The four types of the substrates enable one cycle of sequencing in the same base extension, with the fifth image and the sixth image separately acquired from the same field of view of different cycles of sequencing or the same field of view at different excitation wavelengths of the same cycle of sequencing. As such, the reference image obtained by processing and collecting information from the fifth image and the sixth image is used as a basis for correction, which can facilitate more accurate image correction.

The fifth image and/or the sixth image may be either one image or a plurality of images. In one example, the fifth image is the first image, and the sixth image is the second image. Further, in some specific embodiments, constructing a reference image further comprises utilizing a seventh image and an eighth image. The image to be registered, the fifth image, the sixth image, the seventh image and the eighth image are acquired from the same field of view of one cycle of sequencing, the fifth image, the sixth image, the seventh image and the eighth image corresponding to fields of view during base extensions of four types of bases A, T/U, G and C, respectively. Constructing a reference image further comprises: performing a coarse registration for the seventh image based on the fifth image, comprising: determining an offset of the seventh image relative to the fifth image, and moving the seventh image based on the offset to obtain a seventh image having undergone the coarse registration; performing a coarse registration for the eighth image based on the fifth image, comprising: determining an offset of the eighth image relative to the fifth image, and moving the eighth image based on the offset to obtain an eighth image having undergone the coarse registration; and merging the fifth image with the sixth image having undergone the coarse registration, the seventh image having undergone the coarse registration and the eighth image having undergone the coarse registration to obtain the reference image.

There is no limitation on how the first registration is implemented. For example, Fourier transform can be used to determine the first offset by frequency domain registration. Specifically, for example, the first offset, the offset between the sixth image and the fifth image, the offset between the seventh image and the fifth image and/or the offset between the eighth image and the fifth image can be determined by referring to two-dimensional discrete Fourier transform in phase-only correlation function in Kenji TAKITA et al., IEICE TRANS. FUNDAMENTALS, VOL. E86-A, NO.8 AUGUST 2003. The first registration/coarse registration can reach a precision of 1 pixel. As such, the first offset can be quickly and accurately determined and/or a reference image that facilitates precise correction can be constructed.

In some specific embodiments, the reference image and the image to be registered are binarized images, which facilitates reduction of operation and quick correction.

In one example, both the image to be corrected and the reference image are binarized images. That is, each pixel in the images is either a or b, for example, a is 1 and b is 0, and the pixels marked 1 are brighter or more intense than the pixels marked 0. The reference image is constructed with the images repeat1, repeat2, repeat3 and repeat4 acquired from four base extensions in one cycle of sequencing, and the fifth image and the sixth image are selected from any one, two or three of the images repeat1-4.

In one example, the fifth image is the image repeat1, and the images repeat2, repeat3 and repeat4 are the sixth images. The images repeat2-4 are subjected to a coarse registration successively based on the image repeat1 to obtain images repeat2-4 having undergone the coarse registration respectively; and then the image repeat1 is merged with the images repeat2-4 having undergone the coarse registration to obtain the reference image. The merging image is an overlap spot on the merging image. Based on the size of spots of the corresponding nucleic acid molecules and the resolution of an imaging system, in one example, two spots distanced by no more than 1.5 pixels on two images are set as an overlap spot. Here, the central block of a composite image from images of 4 repeats is used as a reference image, which allows the reference image to have enough spots for subsequent registration, and allows information about spots in the detected and located center block to be relatively more accurate for accurate registration.

In one example, the steps below are followed for image correction: 1) performing a coarse correction for an image repeat5 acquired from a field of view of a base extension of another cycle of sequencing, wherein the image repeat5 is a binarized image, the central area such as 512 × 512 block of the image and a central area of a composite image from images repeat1-4 (an 512 × 512 block at the center of the corresponding reference image) are subjected to two-dimensional discrete Fourier transform to obtain an offset (x0,y0) by frequency domain registration, that is, to complete coarse image registration, with x0 and y0 reaching an precision of 1 pixel; 2) merging the image having undergone the coarse registration with the reference image based on spots in the images, comprising: calculating an offset (xl,yl) of the overlap spots in the central block of the image repeat5 and the corresponding block of the reference image according to the equation offset (xl,yl) = coordinate position of the spot in the image to be corrected - coordinate position of the corresponding spot in the reference image, which can be expressed as offset (xl,yl) = curRepeatPoints - basePoints; and calculating an average offset of all overlap spots to obtain the fine offset ranging from [0,0] to [1,1]. In one example, two spots distanced by no more than 1.5 pixels in two images are set as one overlap spot. 3) To sum up, an offset (x0,y0) - (xl,yl) for different cycles in a field of view (fov) is obtained, which for a spot (peak) can be expressed as: curRepeatPoints + (x0,y0) - (x1,y1), where curRepeatPoints represents the original coordinates of the spot, that is, coordinates in the image before correction. The correction results from the above image correction have high accuracy, and the correction precision is less than or equal to 0.1 pixel. FIG. 4 illustrates the correction process and results. In FIG. 4, an image C is corrected based on an image A, circles in the images A and C represent spots, and spots with the same numerical symbols are overlap spots, and image C->A represents the correction result, that is, the result of aligning the image C to the image A.

The method for identifying and detecting spots on images is not limited. In some specific embodiments, detecting and identifying the spots in images, i.e., detecting signals from extended bases/base clusters in the images, comprises detecting spots for an image using k1 × k2 matrices, determining that the matrix in which a center pixel value of the matrix is not less than any non-center pixel value of the matrix corresponds to a candidate spot, and determining whether the candidate spot is the spot, wherein both k1 and k2 are odd numbers greater than 1, and the k1 × k2 matrix comprises k1 × k2 pixels. The image is, for example, the image to be registered or an image in the construction of the reference image. Spots (or peaks) in an image (particularly an image acquired from a nucleic acid sequence determination) can be detected quickly and effectively by this method. There is no special limitation on the image to be detected, i.e., original input data. The method is applicable to processing and analysis of any images generated by a nucleic acid sequencing platform based on optical detection, including but not limited to next- and third-generation sequencing, and features high accuracy and high efficiency and more information about the sequence acquired from the image. Specifically, for a random image and signal identification requiring high accuracy, the method has special advantages.

In some embodiments, the image is acquired from a nucleic acid sequence determination, and nucleic acid molecules carry optically detectable labels, e.g., fluorescent labels. The fluorescent molecules can be excited under the irradiation of laser with a specific wavelength to emit fluorescence, and the image is acquired by the imaging system. The acquired image contains peaks/spots which may correspond to the positions of the fluorescent molecules. It can be understood that at a focal plane position, the spots corresponding to the positions of the fluorescent molecules in the acquired image are smaller and brighter; and at a non-focal plane position, the spots corresponding to the positions of the fluorescent molecules in the acquired image are larger and darker. In addition, other substances/information which are not targeted or are hard to utilize later, such as impurities, may exist in a field of view; and further, during the photographing of the field of view of a single molecule, a large number of molecule aggregates (clusters) will also interfere with the acquisition of target single molecule information. The single molecule is a few molecules, for example, the number of molecules is not greater than 10, 8, 6, 5 or 3, e.g., 1, 2, 3, 4, 5, 6 or 8.

In some examples, the center pixel value of the matrix is greater than a first preset value, any non-center pixel value of the matrix is greater than a second preset value, and the first preset value and the second preset value are related to an average pixel value of the image.

In some embodiments, the k1 × k2 matrix can be used to perform traversal detection on the image, and the setting of the first preset value and/or the second preset value is related to the average pixel value of the image. For a grayscale image, the pixel value refers to the grayscale value. In the k1 × k2 matrix, k1 and k2 may or may not be equal. In one example, related parameters of an imaging system includes: a 60× magnification for an objective lens, a size of 6.5 µm for an electronic sensor, the smallest identifiable size of 0.1 µm for a microscopic image acquired by the electronic sensor, a 16-bit grayscale or color image of 512 × 512, 1024 × 1024 or 2048 × 2048 for an output or input image, and a range of greater than 1 to less than 10 for k1 and k2. In one example, k1 = k2 = 3; and in another example, k1 = k2 = 5.

In one example, based on a large number of image processing statistics, the first preset value is set as 1.4 times the average pixel value of the image, the second preset value is set as 1.1 times the average pixel value of the image, and thereby, interference can be eliminated and a spot detection result can be obtained from optically detectable labels. Candidate spots can be further screened and determined by size, degree of similarity with an ideal spot, and/or intensity. In some specific embodiments, the size of a candidate spot in the image is quantitatively reflected and compared by the size of connected component corresponding to the candidate spot, so as to screen and determine whether the candidate spot is a spot needed.

In one example, determining whether a candidate spot is a spot comprises: calculating the size of a connected component corresponding to a candidate spot according to the equation Area = A × B, and determining the candidate spot with the size of corresponding connected component greater than a third preset value as the spot, wherein A represents the size of the pixel connectivity in a row where the center of a matrix corresponding to the candidate spot is located, B represents the size of the pixel connectivity in a column where the center of the matrix corresponding to the candidate spot is located, and a pixel connectivity in the k1 × k2 matrix which is greater than an average pixel value is defined as the connected component corresponding to the candidate spot. As such, spots which correspond to labeled molecules and are applicable for subsequent sequence identification can be effectively obtained, so that nucleic acid sequence information can be obtained.

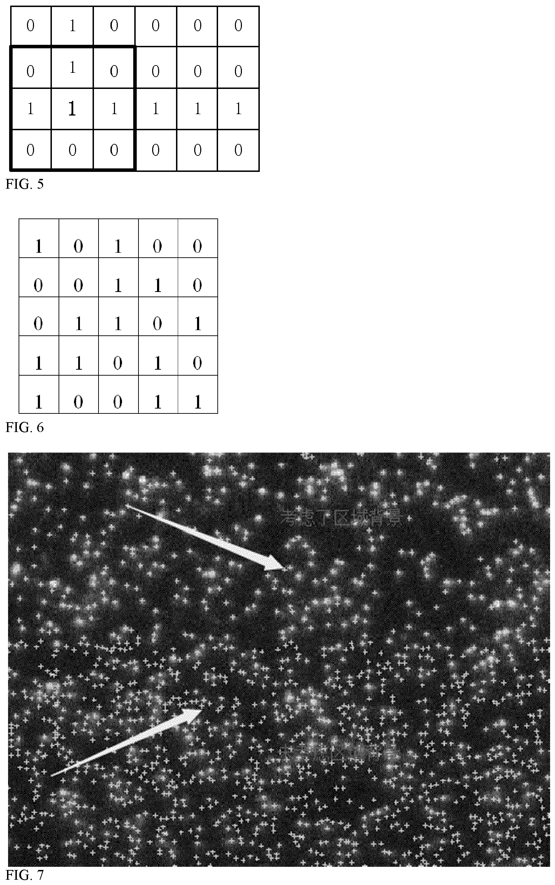

In one example, based on the average pixel value of the image, two or more adjacent pixels which are not less than the average pixel value is the pixel connectivity. As shown in FIG. 5, the bold and enlarged point represents the center of the matrix corresponding to the candidate spot; the thick frame represents the 3 × 3 matrix corresponding to the candidate spot; the pixels marked as 1 are pixels which are not less than the average pixel value of the image; and the pixels marked as 0 are pixels which are less than the average pixel value. It can be seen that A = 3 and B = 6, so the size of the connected component corresponding to the candidate spot is A × B = 3 × 6.

The third preset value can be determined according to the sizes of connected components corresponding to all the candidate spots in the image. For example, by calculating the size of a connected component corresponding to each candidate spot in the image, the average size of the connected components corresponding to the spots, which represents a feature of the image, is taken as a third preset value. For another example, the sizes of the connected components corresponding to the candidate spots in the image can be sorted in ascending order, and the size of the connected component at the 50th, 60th, 70th, 80th or 90th percentile is taken as the third preset value. As such, spot information can be obtained effectively, which is favorable for subsequent nucleic acid sequence identification.

In some examples, the intensities of candidate spots are quantitatively reflected and compared by statistically analyzing setting parameters, so as to screen the candidate spots. In one example, determining whether a candidate spot is a spot comprises: calculating score of a candidate spot according to the equation Score = ((k1 × k2 - 1)CV - EV)/((CV + EV)/(kl × k2)), and determining the candidate spot with the score greater than a fourth preset value as the spot, wherein CV represents the center pixel value of a matrix corresponding to the candidate spot, and EV represents the sum of non-center pixel values of the matrix corresponding to the spot. As such, spots which correspond to labeled molecules and are applicable for subsequent sequence identification can be effectively obtained, so that nucleic acid sequence information can be obtained.

The fourth preset value can be determined according to the scores of all the candidate spots in the image. For example, when the number of the candidate spots in the image is greater than a certain number which meets a statistic requirement for quantity, for example, the number of the candidate spots in the image is greater than 30, the scores of all the candidate spots in the image can be calculated and sorted in ascending order, and the fourth preset value can be set as a score at the 50th, 60th, 70th, 80th or 90th quantile. As such, the candidate spots with scores less than the score at the 50th, 60th, 70th, 80th or 90th quantile can be discarded, so that target spots can be obtained effectively, which is favorable for subsequent accurate base sequence identification. The basis of this processing or screening setting is that generally a concentrated spot with great difference in intensity/pixel value between the center and the edge is considered to be a spot corresponding to the position of a molecule to be detected. In general, the number of candidate spots in an image is greater than 50, 100 or 1000.

In some examples, candidate spots are screened according to morphology and intensity/brightness. In one example, determining whether a candidate spot is a spot comprises: calculating the size of a connected component corresponding to a candidate spot according to the equation Area = A × B, and calculating the score of the candidate spot according to the equation Score = ((k1 × k2 - 1)CV - EV)/((CV + EV)/(kl × k2)), wherein A represents the size of the pixel connectivity in a row where the center of a matrix corresponding to the candidate spot is located, B represents the size of the pixel connectivity in a column where the center of the matrix corresponding to the candidate spot is located, a pixel connectivity in the k1 × k2 matrix which is greater than an average pixel value is defined as the connected component corresponding to the candidate spot, CV represents the center pixel value of a matrix corresponding to the candidate spot, and EV represents the sum of non-center pixel values of the matrix corresponding to the spot; and determining the candidate spot with the size of corresponding connected component greater than a third preset value and the score greater than a fourth present value as the spot. As such, spot information which corresponds to nucleic acid molecules and is favorable for subsequent sequence identification can be obtained effectively. The third preset value and/or the fourth preset value can be considered and set by reference to aforementioned specific embodiments.

In some specific embodiments, identifying and detecting a spot comprises: preprocessing an image to obtain a preprocessed image, wherein the image is selected from at least one of a first image, a second image, a third image, a fourth image, a fifth image, a sixth image, a seventh image and an eighth image; determining a critical value to simplify the preprocessed image so as to obtain a simplified image, comprising: assigning a first preset value to a pixel value of a pixel in the preprocessed image less than the critical value, and assigning a second preset value to a pixel value of a pixel in the preprocessed image not less than the critical value; determining a first spot detection threshold c1 based on the preprocessed image; and identifying a candidate spot in the image based on the preprocessed image and the simplified image, comprising: determining a pixel matrix meeting at least two of the following conditions i)-ii) as the candidate spot: i) in the preprocessed image, the center pixel of the pixel matrix has the maximum pixel value, the pixel matrix is represented by r1 × r2, both r1 and r2 are odd numbers greater than 1, and the pixel matrix r1 × r2 comprises r1 × r2 pixels, ii) in the simplified image, the pixel value of the center pixel of the pixel matrix is the second preset value, and the pixel connectivity in the pixel matrix is greater than (2/3) × r1 × r2, and iii) in the preprocessed image, the pixel value of the center pixel of the pixel matrix is greater than a third preset value, and g1 × g2 is > c1, where g1 is a correlation coefficient of two-dimensional Gaussian distribution in an m1 × m2 area centered on the center pixel of the pixel matrix, g2 is pixels in the m1 × m2 area, both m1 and m2 are odd numbers greater than 1, and the m1 × m2 area comprises m1 × m2 pixels; and determining whether the candidate spot is the spot. Spots in an image (particularly an image acquired from a nucleic acid sequence determination) can be detected quickly and effectively by this method, including using determining conditions or the combination thereof determined by training with a large amount of data. There is no special limitation on the image to be detected, i.e., original input data. The method is applicable to processing and analysis of any images generated by a nucleic acid sequencing platform based on optical detection, including but not limited to next- and third-generation sequencing, and features high accuracy and high efficiency and more information about the sequence acquired from the image. Specifically, for a random image and signal identification requiring high accuracy, the method has special advantages.

For a grayscale image, the pixel value refers to the grayscale value. For a color image having three pixel values for each pixel, the color image may be converted into a grayscale image before detecting the spots, so as to reduce the calculation and complexity in an image detection process. A non-grayscale image may be converted into a grayscale image with methods including but not limited to floating point algorithm, integer method, shift method, mean value method, etc.

In some embodiments, preprocessing the image comprises: determining a background of the image using opening operation; converting the image into a first image based on the background using top-hat operation; applying a Gaussian blur to the first image to obtain a second image; and sharpening the second image to obtain the preprocessed image. In this way, noise of the image can be effectively reduced or the signal-to-noise ratio of the image can be improved, which helps to accurately detect a spot.

Preprocessing can be carried out by referring to the image processing method and system for gene sequencing disclosed in CN107945150A; specifically, opening operation is a morphological process, that is, a process of sequential expansion and corrosion. The corrosion reduces the foreground (a portion of interest), and the expansion enlarges the foreground. The opening operation can be used to eliminate small objects, separate objects at a fine point, and smooth the boundary of a large object without significantly changing its area. In this embodiment, the size of a structural element p1 × p2 (a basic template used to process the image) for opening operation on the image is not specifically defined, and p1 and p2 are odd numbers. In one example, the structural element p1 × p2 may be 15 × 15, 31 × 31, or the like, and finally a preprocessed image that is beneficial for subsequent processing and analysis can be obtained.

The top-hat operation is usually used to separate plaques that are brighter than neighboring points (peaks/spots). When an image has a large area of background and regular small items, the background may be extracted using top-hat operation. In one example, the top-hat transformation of an image comprises: performing the opening operation on the image, and subtracting the opening operation result from the original image to obtain a first image, i.e., the image obtained by the top-hat transformation. The mathematical expression of the top-hat transformation is dst = tophat(src,element) = src - open(src,element). The inventor believes that, the opening operation may enlarge cracks or blocks of low brightness. Therefore, the image obtained by subtracting opening operation result from the original image may highlight blocks brighter than surrounding blocks in the original image. The operation is related to the size of a selected kernel, in other words, related to an expected size of the peak/spot. If the peak has an unexpected size, the processing may lead to many small bumps on the whole image, a specific example of which may be a defocused image, i.e., messed and blurred peaks/spots. In one example, the expected size of the peak, or the size of the kernel, is 3 × 3, and the image acquired from the top-hat transformation is conducive to subsequent denoising process.

Gaussian blur, also referred to as Gaussian filter, is a linear smoothing filter applicable for eliminating Gaussian noise, and is widely used in denoising of image processing. Generally speaking, the Gaussian filter is a process of weighted averaging on the whole image. A value of each pixel is a weighted average of the value itself and other pixel values in neighborhood. The specific procedure of the Gaussian filter is: scanning each pixel in the image using a template (also referred to as convolution or mask), and replacing the value of the center pixel of the template with a weighted average grayscale value of pixels in neighborhood determined using the template. In one example, a Gaussian blur is applied to the first image by using GaussianBlur function in OpenCV. The Gaussian distribution parameter Sigma is 0.9, and the two-dimensional filter matrix (convolution kernel) used is 3 × 3. From a perspective of an image, after the Gaussian blur, the small bumps on the first image are smoothed, and edges of the image are smooth. Further, the second image, i.e., the image acquired from the Gaussian filter, is sharpened, for example, by two-dimensional Laplacian sharpening. From a perspective of an image, edges are sharpened after processing, and the image acquired from the Gaussian blur is restored.

In some embodiments, simplifying the preprocessed image comprises: determining a critical value based on the background and the preprocessed image; and comparing pixel values of pixels in the preprocessed image with the critical value so as to obtain a simplified image, comprising: assigning a first preset value to pixel values of the pixels in the preprocessed image less than the critical value, and assigning a second preset value to the pixel values of the pixels in the preprocessed image not less than the critical value. As such, according to the critical value determining manner and the critical value determined by summarizing a large amount of data, the preprocessed image is simplified, for example, by binarization, which may facilitate subsequent accurate spot detection, accurate base identification, high-quality data acquisition, and the like.

Specifically, in some examples, acquiring a simplified image comprises: dividing the sharpening result obtained after preprocessing by an opening operation result to obtain a set of values corresponding to the pixels of the image; and determining a critical value of the pre-binarized image through this set of values. For example, the set of values may be sorted in ascending order, and the value at the 20th, 30th or 40th percentile in the set of values may serve as the binarization critical value/threshold. As such, the binarized image may facilitate subsequent accurate detection and identification of spots.

In one example, the structural element for opening operation of image preprocessing is p1 × p2, and the preprocessed image (the sharpening result) is divided by the opening operation result to obtain a set of arrays/matrices p1 × p2 of the same size as the structural element. The p1 × p2 values comprised in each array are sorted in ascending order, and the 30th percentile in the array serves as the binarization critical value/threshold of the block (numerical matrix). As such, a threshold is determined for binarization in each block of the image, and the final binarization result highlights required information while denoising, which facilitate subsequent accurate detection of spots.

In some examples, a first spot detection threshold is determined by Otsu's method. The Otsu's method (Otsu's algorithm) can also be referred to as a method of maximum inter-class variance that maximizes the inter-class variance to segment an image, which indicates fewer segmentation errors and high accuracy. It is assumed that the segmentation threshold of the foreground and the background of the preprocessed image is T(c1), the proportion of pixels in the foreground to the whole image is w0 with the average grayscale value being µ0, and the proportion of pixels in the background to the whole image is w1 with the average grayscale value being µ1. The overall average grayscale value of the image to be processed is denoted as µ, and the inter-class variance is denoted as var, which are: *µ* = *ω*₀^{∗}*µ*₀ +*ω*₁^{∗}*µ*₁; var = *ω*₀(*µ*₀ - *µ*)² +*ω*₁ (*µ*₁ - *µ*²). The latter is substituted into the former, getting the following equation: var = *ω*₀*ω*₁(*µ*₁ - *µ*₀)². The segmentation threshold T that maximizes the inter-class variance is acquired by a traversal method, and it is the required first spot detection threshold c1.

In some embodiments, identifying a candidate spot in the image based on the preprocessed image and the simplified image comprises determining a pixel matrix fulfilling at least two of the conditions i)-iii) as the candidate spot. As such, the accuracy of subsequent nucleic acid sequencing based on spot information and the quality of reads may be effectively improved.

Specifically, in one example, conditions required for determining a candidate spot include i), wherein r1 and r2 may or may not be equal. In one example, related parameters of an imaging system includes: a 60× magnification for an objective lens, a size of 6.5 µm for an electronic sensor, the smallest identifiable size of 0.1 µm for a microscopic image acquired by the electronic sensor, a 16-bit grayscale or color image of 512 × 512, 1024 × 1024 or 2048 × 2048 for an output or input image, and a range of greater than 1 to less than 10 for r1 and r2. In one example, in a preprocessed image, r1 and r2 are set at 3 according to the expected size of spot; and in another example, r1 and r2 are set at 5.

In one example, conditions required for determining a candidate spot include ii), namely in the simplified image, the pixel value of the center pixel of the pixel matrix is the second preset value, and the pixel connectivity of the pixel matrix is greater than (2/3) × r1 × r2, i.e., the pixel value of the center pixel is greater than the critical value, and the pixel connectivity is greater than two thirds of the matrix. Herein, two or more adjacent pixels having a pixel value of the second preset value are referred to as connected pixels/pixel connectivity. For example, the simplified image is a binarized image, the first preset value is 0, and the second preset value is 1. As shown in FIG. 5, a bold and enlarged point represents the center of the pixel matrix, and a thick frame represents a 3 × 3 pixel matrix, i.e., r1 = r2 = 3. The pixel value of the center pixel of the matrix is 1, and the pixel connectivity is 4 (less than (2/3) × r1 × r2 = 6). The pixel matrix does not fulfill the condition b), and the spot is not the candidate spot.

In one example, conditions required for determining a candidate spot include iii), namely in the preprocessed image, g2 is corrected pixels in the m1 × m2 area, i.e., a sum of the corrected pixels in the m1 × m2 area. In one example, correction is performed according to the proportion of the pixels with pixel values equal to the second preset value in the corresponding m1 × m2 area in the simplified image. For example, as shown in FIG. 6, m1 = m2 = 5, the proportion of the pixels with pixel values equal to the second preset value in the corresponding m1 × m2 area in the simplified image is 13/25 (13 "1"s), and corrected g2 is 13/25 of original g2. As such, it facilitates accuracy of the spot detection and identification, and subsequent analysis and interpretation of spot information.

In some examples, determining whether a candidate spot is a spot further comprises: determining a second spot detection threshold based on the preprocessed image, and determining a candidate spot with a pixel value not less than the second spot detection threshold as a spot. In one specific example, a pixel value of a position to which the coordinates of the candidate spot correspond is assigned to the pixel value of the candidate spot. After the candidate spots are further screened using the second spot detection threshold determined based on the preprocessed image, at least a part of spots that are more likely the image background or interference but are similar to "spots" in brightness (intensity) and/or shape may be excluded, which helps to accurately identify a sequence based on the spot, and improves quality of reads.

In one example, the coordinates of the candidate spot, including sub-pixel coordinates, may be obtained by centroid method. The pixel value/grayscale value of the coordinates of the candidate spot is calculated by bilinear interpolation.

In some specific examples, determining whether a candidate spot is a spot comprises: dividing the preprocessed image into a set of blocks of a predetermined size, and sorting pixels in the block according to the pixel values to determine the second spot detection threshold corresponding to the block; and determining a candidate spot in the block with a pixel value not less than the second spot detection threshold corresponding to the block as a spot. As such, a difference between different blocks of the image such as an overall difference of light intensity is distinguished, and each spot is further detected and identified, so as to help to accurately identify the spot and find more spots.

When dividing the preprocessed image into a set of blocks of a predetermined size, the blocks may or may not overlap with each other. In one example, the blocks do not overlap with each other. In some embodiments, the size of the preprocessed image is not less than 512 × 512, such as 512 × 512, 1024 × 1024, 1800 × 1800, or 2056 × 2056, and the block of the predetermined size may be set as 200 × 200. As such, the spot is quickly calculated, determined, and identified.

In some embodiments, when determining a second spot detection threshold corresponding to the block, pixel values of pixels in each block are sorted in ascending order. The p10 + (p10 - p1) × 4.1 is used as the second spot detection threshold corresponding to the block, i.e., a background of the block, where p1 represents the first percentile, and p10 represents the tenth percentile. The threshold is a relatively stable threshold determined by training with a large amount of data, which can be adapted to the optical environments of various image acquisitions, including detection of images obtained by different laser powers and/or images with various spot densities. Spots can be screened through the threshold to eliminate a large amount of non-target spots, thereby facilitating subsequent rapid analysis and obtaining accurate results. It can be understood that when overall pixel distribution of the image significantly changes due to the system settings including major adjustments to an optical system, the threshold may need appropriate adjustment. FIG. 7 is a schematic showing the comparison between spot detection results obtained before and after the processing, i.e., a schematic showing spot detection results before and after removing the background of a block. The upper half of FIG. 7 is the spot detection result after the processing, the lower half is the spot detection result before the processing, and the cross marks are candidate spots or spots.

In some specific embodiments, in S2, the coordinates of spots in a spot set corresponding to a sequencing template are aligned with an image to be detected base on a same coordinate system; for example, a spot set corresponding to a sequencing template and an image to be detected are both placed in a coordinate system of the image obtained from the first cycle of sequencing, the coordinates of spots in the spot set corresponding to the sequencing template are marked on the image to be detected, and the coordinates of each spot can be determined by centroid method. As such, the coordinates of corresponding positions in the image to be detected can be quickly and accurately determined.

The method of determining the intensity of a certain position in the image is not limited, and for example, a sub-pixel value/gray-scale value for the position can be calculated by bilinear interpolation, quadratic function interpolation, quadratic spline interpolation, or the like as the intensity for the position. In some specific embodiments, the intensity of a corresponding coordinate position in the image to be detected in S4 may be absolute intensity, for example, a pixel value of the position, or may be relative intensity, for example, a correlation value based on the pixel value of the position, and for example, a related value after performing noise reduction, background removal and/or pixel subtraction using adjacent pixels in the image to be detected.

In some specific embodiments, the preset threshold corresponds to the image to be detected, and one preset threshold corresponds to one or more images to be detected, that is, when the one or more images to be detected are detected using the method for base calling, one preset threshold can be shared. In such cases, the background intensity of a corresponding coordinate position in the image to be detected is relative intensity; for example, the relative intensity is determined according to absolute intensity of the corresponding coordinate position and background intensity of the block that the corresponding coordinate position is in. The block that the corresponding coordinate position is in is a block comprising the position, preferably, a block that comprises x1 ×y1 pixels and does not need to be strictly centered on the position, where x1 and y1 are both natural numbers, x1 ×y1 is not less than 100, preferably, not less than 1000. As such, a base can be quickly and accurately called.

In some examples, the background intensity of the block that the corresponding coordinate position is in is determined by the following steps: sorting the pixels in an x1 ×y1 block that the corresponding coordinate position is in according to the pixel values to obtain a distribution curve of the number of the pixels in the x1 ×y1 block; and determining the background intensity of the block that the corresponding coordinate position is in based on the distribution curve. The sorting may be ascending sorting or descending sorting, and the ascending sorting result is used as an example below. Those skilled in the art can obtain a curve by descending sorting and determine related parameters or conditions according to the curve by the example, and can also calculate the intensity of the block.

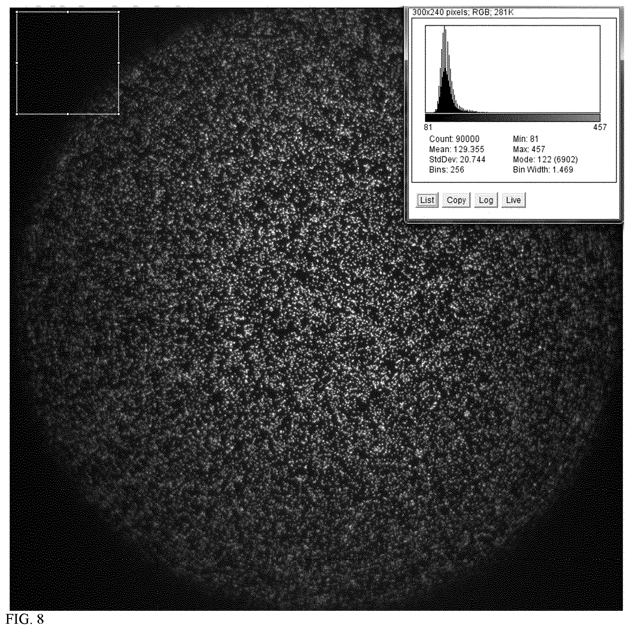

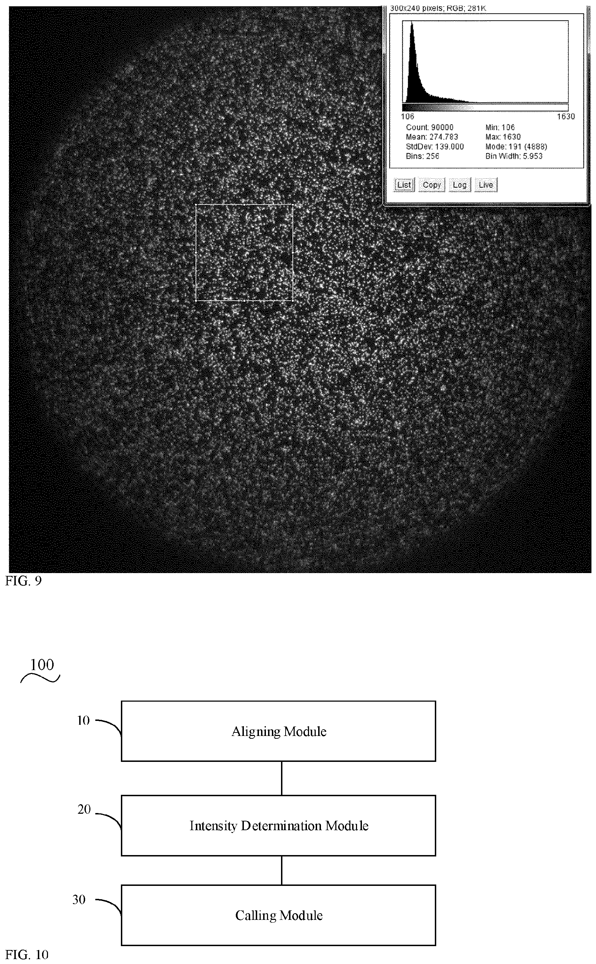

In some examples, the size of the image to be detected is 1800 × 1800, and x1 = y1 = 300. FIGs. 8 and 9 show a distribution curve (histogram) of a 300 × 300 block, i.e., ninety thousand pixels sorted in ascending order according to the pixel values, with the abscissa representing pixels (pixel values) and the ordinate representing the number of pixels. FIGs. 9 and 10 are taken from 300 × 300 blocks (indicated by boxes) at the edge of the field of view and the center of the field of view, respectively, in the same image. It can be seen that the number of pixels in the background of the image is symmetrically distributed along with the variation of the pixel values, which accords with normal distribution or approximately accords with normal distribution, as shown in FIG. 8; the overlap between the background and the spots makes the curve/histogram show a tendency to broadened distribution, a relatively left shifted peak, and slow decline on the right side, i.e., a tail on the right side, as shown in FIG. 9. In addition, other interference spots present in the block, such as abnormal intensity or large density difference in the spot distribution, cause the curve to be asymmetrically distributed and lead to abnormal bulges or depressions at the peak or right trough or near the peak or trough (not according to the original tendency).

In one example, the highest-frequency pixel value, i.e. the peak, of the distribution curve is taken as the background intensity of the block that the corresponding coordinate position is in. Taking the relatively stable highest-frequency pixel value as the background intensity of the corresponding block helps to quickly, simply and accurately perform subsequent base calling. The method of estimating or determining the background intensity of the block in the this embodiment is not limited; for example, the opening operation of OpenCV or the like may be used.

In some other specific embodiments, after induction, testing and verification based on a large amount of image data, the inventor develops a formula for determining the peak pixel value of the distribution curve, i.e. the intensity *I*_{bock} of the block that a corresponding coordinate position is in, *I*_{bock} = *I*ⱼ₁ + (*I*ⱼ₂ - *I*ⱼ₃) × t1, where *I*ⱼ₁, *I*ⱼ₂ and *I*ⱼ₃ are the pixel values corresponding to the j1th percentile, j2th percentile and j3th percentile, respectively, on the curve; j1, j2 and j3 are all integers less than 50 and not less than 1; j2 > 8 + j3; and t1 is a first correction coefficient and determined by j 1, j2 and j3. The peak pixel value estimated by the formula is relatively reliable, and the formula is suitable for images generated by various sequencing platforms, in particular to images generated by a single-molecule sequencing platform.

In some examples, preferably, j1 is selected from [1, 40], j2 is selected from [6, 40], j3 is selected from [1, 30], and 40 < j1 + (j2 - j3) × t1 < 50; as such, the peak pixel value favorable for accurate base calling can be accurately estimated.

Further, the background intensity of the block that the corresponding coordinate position is in is determined according to the above formula, the intensity of the corresponding coordinate position in the image to be detected is the ratio of the absolute intensity of the corresponding coordinate position to the background intensity of the block that the corresponding coordinate position is in, and the preset threshold is selected from [0.85, 0.95], so as to perform S6 to compare the intensity of the corresponding coordinate position in the image to be detected with the preset threshold, thereby performing base calling based on the information of the position in the image to be detected where the intensity is greater than the preset threshold. As such, bases can be accurately called without losing too much information. In one specific example, j1 = j2 = 10, j3 = 1, t1 = 4.1, and the preset threshold is 0.9, which are better suitable for base calling based on images generated by a single-molecule sequencing platform.

In some other specific embodiments, the preset threshold corresponds to the corresponding coordinate position in the image to be detected, that is, the preset threshold generally varies with the position, and one preset threshold corresponds to one corresponding coordinate position in the image to be detected. In these cases, the intensity of the corresponding coordinate position in the image to be detected is absolute intensity, for example, the pixel value of the position.

The preset threshold corresponding to each position can be determined in S6, or can be determined and saved in advance. In some examples, the preset threshold is determined in relation to the background intensity of the block that the corresponding coordinate position is in, and the block that the corresponding coordinate position is in is a block comprising the position on the image to be detected, and is a block that comprises x2×y2 pixels and does not need to be strictly centered on the position, where x2 and y2 are both natural numbers, x2×y2 is not less than 100, preferably, not less than 1000. As such, the preset threshold can be quickly and effectively determined, and bases can be accurately called.

Specifically, in some examples, determining the preset threshold comprises: sorting the pixels in an x2×y2 block that the corresponding coordinate position is in according to the pixel values to obtain a distribution curve of the number of the pixels in the x2×y2 block; and determining the preset threshold based on the distribution curve. The sorting may be ascending sorting or descending sorting, and the ascending sorting result is used as an example below. Those skilled in the art can obtain a curve by descending sorting and determine related parameters or conditions according to the curve by the example, and can also obtain the preset threshold by calculation.

Referring to FIGs. 8 and 9, the right trough pixel value of the distribution curve is used as the preset threshold, so that the confidence level of the accuracy of the identified base can be expected to reach 90%, 95% or 99% or more, which is favorable for subsequent accurate base calling.

The method of determining the trough pixel value of the curve is not limited. In some other specific embodiments, after induction, testing and verification based on a large amount of image data, the inventor develops a formula for determining the right trough pixel value of such distribution curves, i.e. the preset threshold, Threshold = *I*ⱼ₄ + (*I*ⱼ₅ - *I*ⱼ₆) × t2, where *I*ⱼ₄, *I*ⱼ₅ and *I*ⱼ₆ are the pixel values corresponding to the j4th percentile, j5th percentile and j6th percentile, respectively; j4, j5 and j6 are all integers less than 50 and not less than 1; j5 > 8 + j6; and t2 is a second correction coefficient and determined by j4, j5 and j6. The right trough pixel value estimated by the formula is relatively reliable, and the formula is suitable for images generated by various sequencing platforms, including images with uniform or non-uniform spot distribution, in particular to images generated by a single-molecule sequencing platform.

Preferably, j4 is selected from [1, 40], j5 is selected from [6, 40], j6 is selected from [1, 30], and 85 < j4 + (j5 - j6) × t2 < 100; as such, the right trough pixel value can be accurately estimated, and accurate base calling can be performed. In one specific example, j4 = j5 = 10, j6 = 1, t2 = 3.7, and base calling is performed based on positions where the intensity is greater than the preset threshold, which are better suitable for images generated by a single-molecule sequencing platform.

In some examples, in addition to the comparison with the preset threshold, the morphology of (the bright spot of) the corresponding position in the image to be detected may be further taken into consideration to screen and determine the position, so that the information of the determined position can objectively reflect whether base extension actually occurs in the position, thereby facilitating accurate base calling.

Logic and/or steps shown in the flowcharts or described herein in other manners, for example, may be considered as a program list of executable instructions that are used to implement logical functions, and may be specifically implemented on any computer-readable storage medium, for an instruction execution system, device, or apparatus (for example, a computer-based system, a system including a processor, or another system that can fetch instructions from the instruction execution system, apparatus, or device and execute the instructions), or for a combination of the instruction execution system, device or apparatus.

In one embodiment, provided is a computer-readable storage medium for storing a program executable by a computer, wherein executing the program comprises implementing the method for base calling according to any of the aforementioned embodiments. The computer-readable storage medium includes, but is not limited to, read-only memories, random access memories, magnetic disks, optical disks, or the like. The computer-readable storage medium may be any device that may include, store, communicate, propagate, or transmit a program for an instruction execution system, device, or apparatus, or for a combination of the instruction execution system, device, or apparatus. More specific examples (this list is not exhaustive) of the computer-readable storage medium include the following: an electrical connection (an electrical device) with one or more buses, a portable computer cartridge (an magnetic device), a random-access memory (RAM), a read-only memory (ROM), an erasable programmable read-only memory (EPROM or flash memory), an optical fiber device, and a portable compact disc read-only memory (CDROM). In addition, the computer-readable storage medium may even be a piece of paper on which the programs can be printed or any other appropriate media, because, for example, the paper or the media may be optically scanned, and the program may be electrically acquired by processing such as edition, decoding, or any other appropriate means when necessary and then stored in a computer storage. The above description of the technical features and advantages of the method for base calling according to any of the embodiments is also applicable to the computer-readable storage medium, and will not be repeated herein.

In one embodiment of the present application, referring to FIG. 10, also provided is a system 100 for base calling for implementing the method for base calling according to any of the aforementioned embodiments of the present application, the system comprising: a aligning module 10 for aligning the coordinates of spots in a spot set corresponding to a sequencing template with an image to be detected so as to determine a corresponding coordinate position in the image to be detected, an intensity determination module 20 for calculating the intensity of the corresponding coordinate position in the image to be detected from the aligning module 10, and a calling module 30 for comparing the intensity of the corresponding coordinate position in the image to be detected from the intensity determination module 20 with a preset threshold and performing base calling based on the information of the position in the image to be detected where the intensity is greater than the preset threshold, wherein the spot set of a corresponding sequencing template is acquired by construction based on a plurality of images; the images and the image to be detected are all acquired from base extensions and correspond to the same field of view; and a plurality of molecules carrying an optically detectable label are present in the field of view during the base extensions, and at least some of the nucleic acid molecules are present as spots in the images and/or the image to be detected.

The above description of the technical features and advantages of the method for base calling according to any of the embodiments is also applicable to the system for base calling in this embodiment of the present application, and will not be repeated herein. For example, in some examples, further included is a template construction module 12, which is connected to the aligning module 10 and configured for constructing a spot set corresponding to a sequencing template based on a plurality of images. The images include a first image, a second image, a third image and a fourth image corresponding to the same field of view during base extensions of four types of bases A, T/U, G and C, respectively. The first image comprises an image M1 and an image M2, the second image comprises an image N1 and an image N2, the third image comprises an image P1 and an image P2, and the fourth image comprises an image Q1 and an image Q2. Sequential and/or simultaneous completion of the base extensions of the four types of bases once is defined as one cycle of sequencing. The images M1 and M2 separately come from two cycles of sequencing, the images N1 and N2 separately come from two cycles of sequencing, the images P1 and P2 separately come from two cycles of sequencing, and the images Q1 and Q2 separately come from two cycles of sequencing. The following steps are performed in the template construction module 12: merging spots in the first image, the second image, the third image and the forth image, recording the number of spots in the same position, and removing spots in a position with a number of 1 to obtain the spot set corresponding to the sequencing template.

Specifically, in the template construction module 12, the merging spots in the first image, the second image, the third image and the forth image comprises: (a) merging spots in the image N1 in the image M1 to obtain a one-time merging image M1, marking overlap spots in the image M1 as A and marking non-overlap spots as B, wherein a plurality of spots distanced by less than a first predetermined pixel in the one-time merging image M1 are counted as one overlap spot; (b) replacing the image N1 with the image PI, the image Q1, the image M2, the image N2, the image P2 or the image Q2, replacing the image M1 with the one-time merging image M1, and repeating (a) multiple times until the spots in all of the images are merged to obtain an original spot set; and (c) removing the spots marked as B in the original spot set to obtain a spot set corresponding to the sequencing template.

In some examples, the images are registered images. The system 100 further comprises a registration module 14 connected to the template construction module 12 and configured for implementing the following steps to register an image: performing a first registration for an image to be registered based on a reference image, wherein the reference image and the image to be registered correspond to the same field of view, comprising: determining a first offset between a predetermined block in the image to be registered and a corresponding predetermined block in the reference image, and moving all spots in the image to be registered based on the first offset to obtain an image to be registered having undergone the first registration; performing a second registration for the image to be registered having undergone the first registration based on the reference image, comprising: merging the image to be registered having undergone the first registration with the reference image to obtain a merging image, calculating an offset of all second overlap spots in a predetermined block in the merging image to determine a second offset, with a plurality of spots distanced by less than a second predetermined pixel defined as a second overlap spot, and moving all spots in the image to be registered having undergone the first registration based on the second offset to register the image to be registered.

In some examples, the registration module 14 comprises a reference image construction module 142 configured for implementing the following steps to construct a reference image: acquiring a fifth image and a sixth image, wherein the fifth image and the sixth image correspond to the same field as the image to be registered; performing a coarse registration for the sixth image based on the fifth image, comprising: determining an offset of the sixth image relative to the fifth image, and moving the sixth image based on the offset to obtain a sixth image having undergone the coarse registration; and merging the fifth image with the sixth image having undergone the coarse registration to obtain the reference image.

Further, in the reference image construction module 142, constructing a reference image further comprises utilizing a seventh image and an eighth image, wherein the fifth image, the sixth image, the seventh image and the eighth image correspond to the same field of view, and the fifth image, the sixth image, the seventh image and the eighth image correspond to fields of view during base extensions of four types of bases A, T/U, G and C, respectively. Constructing a reference image further comprises: performing a coarse registration for the seventh image based on the fifth image, comprising: determining an offset of the seventh image relative to the fifth image, and moving the seventh image based on the offset to obtain a seventh image having undergone the coarse registration; performing a coarse registration for the eighth image based on the fifth image, comprising: determining an offset of the eighth image relative to the fifth image, and moving the eighth image based on the offset to obtain an eighth image having undergone the coarse registration; and merging the fifth image with the sixth image having undergone the coarse registration, the seventh image having undergone the coarse registration and the eighth image having undergone the coarse registration to obtain the reference image.

In some examples, the reference image and the image to be registered are both binarized images.

In some examples, two-dimensional discrete Fourier transform is employed to determine the first offset, the offset of the sixth image relative to the fifth image, the offset of the seventh image relative to the fifth image, and/or the offset of the eighth image relative to the fifth image.

In some examples, the system 100 further comprises a spot detection module 16 connected to the aligning module 10, the template construction module 12 and/or the registration module 14 and configured for implementing the following steps to detect spots in an image: preprocessing the image to obtain a preprocessed image; determining a critical value to simplify the preprocessed image so as to obtain a binarized image, comprising: assigning a first preset value to a pixel value of a pixel in the preprocessed image less than the critical value, and assigning a second preset value to a pixel value of a pixel in the preprocessed image not less than the critical value; determining a first spot detection threshold c1 based on the preprocessed image; and identifying the spots in the image based on the preprocessed image and the binarized image, comprising: determining a pixel matrix meeting at least two of the following conditions i)-iii) as a candidate spot: i) in the preprocessed image, the center pixel of the pixel matrix has the maximum pixel value, the pixel matrix is represented by k1 × k2, both k1 and r2 are odd numbers greater than 1, and the pixel matrix k1 × k2 comprises k1 × k2 pixels, ii) in the binarized image, the pixel value of the center pixel of the pixel matrix is the second preset value, and the pixel connectivity in the pixel matrix is greater than (2/3) × k1 × k2, and iii) in the preprocessed image, the pixel value of the center pixel of the pixel matrix is greater than a third preset value, and g1 × g2 is > c1, where g1 is a correlation coefficient of two-dimensional Gaussian distribution in an m1 × m2 area centered on the center pixel of the pixel matrix, g2 is pixels in the m1 × m2 area, both m1 and m2 are odd numbers greater than 1, and the m1 × m2 area comprises m1 × m2 pixels.

Further, in the spot detection module 16, the following steps are implemented to determine whether the candidate spot is a spot: determining a second spot detection threshold based on the preprocessed image, comparing the pixel value of the candidate spot with the second spot detection threshold, determining a candidate spot with a pixel value not less than the second spot detection threshold as a spot, and taking the pixel value of the position indicated by the coordinates of the candidate spot as the pixel value of the candidate spot.

Specifically, in some examples, in the spot detection module 16, determining whether a candidate spot is a spot comprises: dividing the preprocessed image into a set of blocks of a predetermined size; sorting pixels in the block according to the pixel values to determine the second spot detection threshold corresponding to the block; and comparing the pixel values of the candidate spots in the block with the second spot detection threshold, and determining a candidate spot in the block with a pixel value not less than the second spot detection threshold corresponding to the block as a spot.

In some examples, the preprocessing the image comprises: determining a background of the image using opening operation; converting the image based on the background using top-hat operation; applying a Gaussian blur to the converted image; and sharpening the Gaussian blurred image to obtain the preprocessed image.

In some examples, the determining a critical value to simplify the preprocessed image so as to obtain a binarized image comprises: determining the critical value based on the background and the preprocessed image, and comparing pixel values of pixels in the preprocessed image with the critical value so as to obtain a binarized image.

In some examples, g2 is a corrected pixel value in the m1 × m2 area, and pixels in the m1 × m2 area are corrected based on the proportion of pixels of the second preset value in the corresponding m1 × m2 area in the binarized image to obtain the corrected pixels in the m1 × m2 area.

In some examples, one preset threshold corresponds to one or more images to be detected. In this case, the intensity of a corresponding coordinate position in the image to be detected is relative intensity; for example, the relative intensity is determined according to absolute intensity of the corresponding coordinate position and background intensity of the block that the corresponding coordinate position is in.

In some examples, in the intensity determination module 20, determining the background intensity of the block that the corresponding coordinate position is in comprises: sorting the pixels in an x1 × y1 block that the corresponding coordinate position is in according to the pixel values to obtain a distribution curve of the number of the pixels in the x1 × y1 block, where x1 and y1 are both natural numbers, and x1 × y1 is not less than 100; and determining the background intensity of the block that the corresponding coordinate position is in based on the distribution curve. Specifically, the sorting is ascending sorting, and the peak pixel value of the distribution curve is used as the background intensity of the block that the corresponding coordinate position is in; the peak pixel value of the distribution curve is estimated according to the formula *I*_{bock} = *I*ⱼ₁ + (*I*ⱼ₂ - *I*ⱼ₃) × t1, where *I*ⱼ₁, *I*ⱼ₂ and *I*ⱼ₃ are the pixel values corresponding to the j1th percentile, j2th percentile and j3th percentile, respectively; j1, j2 and j3 are all integers less than 50 and not less than 1; j2 > 8 + j3; and t1 is a first correction coefficient and determined by j 1, j2 and j3.

Further, j1 is selected from [1, 40], j2 is selected from [6, 40], j3 is selected from [1, 30], and 40 < j1 + (j2 - j3) × t1 < 50. Accordingly, the preset threshold is selected from any value in [0.85, 0.95].

In some other examples, one preset threshold corresponds to a corresponding coordinate position in one images to be detected. In such cases, the intensity of the corresponding coordinate position in the image to be detected is absolute intensity.

In some examples, the system 100 further comprises a threshold determination module 40 connected to the calling module 30 and configured for implementing the following steps to determine the preset threshold: sorting the pixels in an x2 × y2 block that the corresponding coordinate position is in according to the pixel values to obtain a distribution curve of the number of the pixels in the x2 × y2 block, where x2 and y2 are both natural numbers, and x2 × y2 is not less than 100; and determining the preset threshold based on the distribution curve.

Specifically, the sorting is ascending sorting, and the right trough pixel value of the distribution curve are used as the preset threshold; the right trough pixel value is estimated according to the formula Threshold = *I*ⱼ₄ + (*I*ⱼ₅ - *I*ⱼ₆) × t2, where *I*ⱼ₄, *I*ⱼ₅ and *I*ⱼ₆ are the pixel values corresponding to the j4th percentile, j5th percentile and j6th percentile, respectively; j4, j5 and j6 are all integers less than 50 and not less than 1; j5 > 8 + j6; and t2 is a second correction coefficient and determined by j4, j5 and j6.

Further, j4 is selected from [1, 40], j5 is selected from [6, 40], j6 is selected from [1, 30], and 85 < j4 + (j5 - j6) × t2 < 100.

In another embodiment, provided is a system for base calling, which comprises: a memory for storing data including a computer-executable program, a processor for executing the computer-executable program to implement the method for base calling according to any of the aforementioned embodiments of the present application. The system is configured for implementing the method for base calling according to any of the aforementioned specific embodiments, and the above description of the technical features and advantages of the method for base calling according to any of the embodiments is also applicable to the system for base calling, and will not be repeated herein. In one embodiment, provided is a sequencing system, which comprises the system for base calling according to any of the aforementioned embodiments.

In one embodiment, provided is a computer program product comprising an instruction, wherein the instruction causes a computer to execute the method for base calling according to any of the aforementioned embodiments of the present application when the program is executed by the computer. The above description of the technical features and advantages of the method for base calling according to any of the embodiments is also applicable to the computer program product, and will not be repeated herein.

In one embodiment, provided is a sequencing system comprising the computer program product according to any of the aforementioned embodiments of the present application. The above description of the technical features and advantages of the method for base calling and/or the computer program product according to any of the embodiments is also applicable to the sequencing system, and will not be repeated herein.

It will be understood by those skilled in the art know that, in addition to implementing the controller/processor in a form of computer-readable program code, same functions can be implemented in a form of a logic gate, a switch, an application-specific integrated circuit, an editable logic controller, an embedded microcontroller, and the like by logically programming the steps. Therefore, the controller/processor may be regarded as a hardware component, and a device included in the controller/processor for implementing various functions may also be regarded as a structure in the hardware component. Alternatively, a device for implementing various functions may be regarded as both a software module for implementing the method and a structure in the hardware component.

In the specification, descriptions such as "one embodiment", "some embodiments", "one or some specific embodiments", "one or some examples", "example" or the like, means that a particular feature, structure or characteristic described in reference to the embodiment or example is included in at least one embodiment or example of the present application.

In the specification, the schematic description of the aforementioned terms do not necessarily refer to the same embodiment or example. Moreover, the specific features, structures and other characteristics described may be combined in any one or more embodiments or examples in an appropriate manner. Although the embodiments of the present application have been illustrated and described, it can be understood by those of ordinary skill in the art that various changes, modifications, replacements and variations can be made to these embodiments without departing from the principle and purpose of the present application, and the scope of the present application is defined by the claims and equivalents therefore.

## Claims

1. A method for base calling, comprising:
aligning the coordinates of spots in a spot set corresponding to a sequencing template with an image to be detected so as to determine a corresponding coordinate position in the image to be detected;
determining the intensity of the corresponding coordinate position in the image to be detected; and
comparing the intensity of the corresponding coordinate position in the image to be detected with a preset threshold, and performing base calling based on the information of the position in the image to be detected where the intensity is greater than the preset threshold; wherein
the spot set corresponding to the sequencing template is acquired by construction based on a plurality of images; the images and the image to be detected are all acquired from base extensions and correspond to the same field of view;
and a plurality of molecules carrying an optically detectable label are present in the field of view during the base extensions, and at least some of the nucleic acid molecules are present as spots in the images and/or the image to be detected.

2. The method according to claim 1, wherein one preset threshold corresponds to one or more images to be detected.

3. The method according to claim 2, wherein the intensity of the corresponding coordinate position in the image to be detected is relative intensity, and the relative intensity is determined according to absolute intensity of the corresponding coordinate position and background intensity of the block that the corresponding coordinate position is in.

4. The method according to claim 3, wherein determining the background intensity of the block that the corresponding coordinate position is in comprises:
sorting pixels in an x1 × y1 block that the corresponding coordinate position is in according to the pixel values to obtain a distribution curve of the number of the pixels in the x1 × y1 block, where x1 and y1 are both natural numbers, and x1 × y1 is not less than 100; and
determining the background intensity of the block that the corresponding coordinate position is in based on the distribution curve.

5. The method according to claim 4, wherein the sorting is ascending sorting, and the peak pixel value of the distribution curve is used as the background intensity of the block that the corresponding coordinate position is in; the peak pixel value of the distribution curve is estimated according to the formula *I*_{block} = *I*ⱼ₁ + (*I*ⱼ₂ - *I*ⱼ₃) × t1, where *I*ⱼ₁, *I*ⱼ₂ and *I*ⱼ₃ are the pixel values corresponding to the j 1th percentile, j2th percentile and j3th percentile, respectively; j 1, j2 and j3 are all integers less than 50 and not less than 1; j2 > 8 + j3; and t1 is a first correction coefficient and determined by j 1, j2 and j3.

6. The method according to claim 5, wherein j1 is selected from [1, 40], j2 is selected from [6, 40], j3 is selected from [1, 30], and 40 < j1 + (j2 - j3) × t1 < 50.

7. The method according to claim 6, wherein the preset threshold is selected from [0.85, 0.95].

8. The method according to claim 1, wherein one preset threshold corresponds to a corresponding coordinate position in one image to be detected.

9. The method according to claim 8, wherein the intensity of the corresponding coordinate position in the image to be detected is absolute intensity.

10. The method according to claim 9, wherein determining the preset threshold comprises:
sorting pixels in an x2 × y2 block that the corresponding coordinate position is in according to the pixel values to obtain a distribution curve of the number of the pixels in the x2 × y2 block, where x2 and y2 are both natural numbers, and x2 × y2 is not less than 100; and
determining the preset threshold based on the distribution curve.

11. The method according to claim 10, wherein the sorting is ascending sorting, and the right trough pixel value of the distribution curve is used as the preset threshold; the right trough pixel value of the distribution curve is estimated according to the formula Threshold = *I*ⱼ₄ + (*I*ⱼ₅ - *I*ⱼ₆) × t2, where
*I*ⱼ₄, *I*ⱼ₅ and *I*ⱼ₆ are the pixel values corresponding to the j4th percentile, j5th percentile and j6th percentile, respectively;
j4, j5 and j6 are all integers less than 50 and not less than 1; j5 > 8 + j6; and t2 is a second correction coefficient and determined by j4, j5 and j6.

12. The method according to claim 11, wherein j4 is selected from [1, 40], j5 is selected from [6, 40], j6 is selected from [1, 30], and 85 < j4 + (j5 - j6) × t2 < 100.

13. The method according to any of claims 1 to 12, wherein the images include a first image, a second image, a third image and a fourth image corresponding to the same field of view during base extensions of four types of bases A, T/U, G and C, respectively; the first image comprises an image M1 and an image M2, the second image comprises an image N1 and an image N2, the third image comprises an image P1 and an image P2, and the fourth image comprises an image Q1 and an image Q2; and sequential and/or simultaneous completion of the base extensions of the four types of bases once is defined as one cycle of sequencing;
the images M1 and M2 separately come from two cycles of sequencing, the images N1 and N2 separately come from two cycles of sequencing, the images P1 and P2 separately come from two cycles of sequencing, and the images Q1 and Q2 separately come from two cycles of sequencing;
constructing the spot set corresponding to the sequencing template based on a plurality of images comprises:
merging spots in the first image, the second image, the third image and the forth image, recording the number of spots in the same position, and removing spots in a position with a number of 1 to obtain the spot set corresponding to the sequencing template.

14. The method according to claim 13, wherein the merging spots in the first image, the second image, the third image and the forth image comprises:
(a) merging spots in the image N1 in the image M1 to obtain a one-time merging image M1, marking overlap spots in the image M1 as A and marking non-overlap spots as B, wherein a plurality of spots distanced by less than a first predetermined pixel in the one-time merging image M1 are counted as one overlap spot;
(b) replacing the image N1 with the image PI, the image Q1, the image M2, the image N2, the image P2 or the image Q2, replacing the image M1 with the one-time merging image M1, and repeating (a) multiple times until the spots in all of the images are merged to obtain an original spot set; and
(c) removing the spots marked as B in the original spot set to obtain a spot set corresponding to the sequencing template.

15. The method according to claim 13, wherein the images are registered images.

16. The method according to claim 15, wherein registering the images comprises:
performing a first registration for an image to be registered based on a reference image, wherein the reference image and the image to be registered correspond to the same field of view, comprising:
determining a first offset between a predetermined block in the image to be registered and a corresponding predetermined block in the reference image, and moving all spots in the image to be registered based on the first offset to obtain an image to be registered having undergone the first registration; and
performing a second registration for the image to be registered having undergone the first registration based on the reference image, comprising:
merging the image to be registered having undergone the first registration with the reference image to obtain a merging image;
calculating an offset of second overlap spots in a predetermined block in the merging image to determine a second offset, with a plurality of spots distanced by less than a second predetermined pixel in the merging image defined as a second overlap spot; and
moving all spots in the image to be registered having undergone the first registration based on the second offset to register the image to be registered.

17. The method according to claim 16, wherein the reference image is acquired by construction, and constructing the reference image comprises:
acquiring a fifth image and a sixth image, wherein the fifth image and the sixth image correspond to the same field of view as the image to be registered;
performing a coarse registration for the sixth image based on the fifth image, comprising: determining an offset of the sixth image relative to the fifth image, and moving the sixth image based on the offset to obtain a sixth image having undergone the coarse registration; and
merging the fifth image with the sixth image having undergone the coarse registration to obtain the reference image.

18. The method according to claim 17, wherein constructing the reference image further comprises: utilizing a seventh image and an eighth image, wherein the fifth image, the sixth image, the seventh image and the eighth image correspond to the same field of view, and the fifth image, the sixth image, the seventh image and the eighth image correspond to fields of view during base extensions of four types of bases A, T/U, G and C, respectively; and constructing the reference image further comprises:
performing a coarse registration for the seventh image based on the fifth image, comprising: determining an offset of the seventh image relative to the fifth image, and moving the seventh image based on the offset to obtain a seventh image having undergone the coarse registration;
performing a coarse registration for the eighth image based on the fifth image, comprising: determining an offset of the eighth image relative to the fifth image, and moving the eighth image based on the offset to obtain an eighth image having undergone the coarse registration; and
merging the fifth image with the sixth image having undergone the coarse registration, the seventh image having undergone the coarse registration and the eighth image having undergone the coarse registration to obtain the reference image.

19. The method according to any of claims 16 to 18, wherein the reference image and the image to be registered are binarized images.

20. The method according to any of claims 16 to 19, wherein two-dimensional discrete Fourier transform is employed to determine the first offset, the offset of the sixth image relative to the fifth image, the offset of the seventh image relative to the fifth image and/or the offset of the eighth image relative to the fifth image.

21. The method according to any of claims 13 to 20, wherein detecting spots in the image comprises:
preprocessing the image to obtain a preprocessed image;
determining a critical value to simplify the preprocessed image so as to obtain a binarized image, comprising:
assigning a first preset value to a pixel value of a pixel in the preprocessed image less than the critical value, and
assigning a second preset value to a pixel value of a pixel in the preprocessed image not less than the critical value;
determining a first spot detection threshold c1 based on the preprocessed image; and
identifying the spots in the image based on the preprocessed image and the binarized image, comprising: determining a pixel matrix fulfilling at least two of the following conditions i)-iii) as a candidate spot:
i) in the preprocessed image, a center pixel of the pixel matrix has the maximum pixel value, the pixel matrix is represented by r1 × r2, both r1 and r2 are odd numbers greater than 1, and the pixel matrix r1 × r2 comprises r1 × r2 pixels;
ii) in the binarized image, the pixel value of the center pixel of the pixel matrix is the second preset value, and the pixel connectivity in the pixel matrix is greater than (2/3) × r1 × r2; and
iii) in the preprocessed image, the pixel value of the center pixel of the pixel matrix is greater than a third preset value, and g1 × g2 is > c1, where g1 is a correlation coefficient of two-dimensional Gaussian distribution in an m1 × m2 area centered on the center pixel of the pixel matrix, g2 is pixels in the m1 × m2 area, both m1 and m2 are odd numbers greater than 1, and the m1 × m2 area comprises m1 × m2 pixels.

22. The method according to claim 21, further comprising: determining whether the candidate spot is a spot, comprising:
determining a second spot detection threshold based on the preprocessed image, and
comparing the pixel value of the candidate spot with the second spot detection threshold, determining a candidate spot with a pixel value not less than the second spot detection threshold as a spot, and taking the pixel value of the pixel indicated by the coordinates of the candidate spot as the pixel value of the candidate spot.

23. The method according to claim 22, wherein determining whether the candidate spot is a spot comprises:
dividing the preprocessed image into a set of blocks of a predetermined size;
sorting pixels in the block according to the pixel values to determine the second spot detection threshold corresponding to the block; and
comparing the pixel value of the candidate spot in the block with the second spot detection threshold, and
determining a candidate spot in the block with a pixel value not less than the second spot detection threshold corresponding to the block as a spot.

24. The method according to any of claims 21 to 23, wherein the preprocessing the image comprises:
determining a background of the image using opening operation;
converting the image based on the background using top-hat operation;
applying a Gaussian blur to the converted image; and
sharpening the Gaussian blurred image to obtain the preprocessed image.

25. The method according to any of claims 21 to 24, wherein the determining a critical value to simplify the preprocessed image so as to obtain a binarized image comprises:
determining the critical value based on the background and the preprocessed image; and
comparing a pixel value of a pixel in the preprocessed image with the critical value so as to obtain the binarized image.

26. The method according to any of claims 21 to 25, wherein g2 is a corrected pixel value in the m1 × m2 area, and pixels in the m1 × m2 area are corrected based on the proportion of pixels of the second preset value in the corresponding m1 × m2 area in the binarized image to obtain the corrected pixels in the m1 × m2 area.

27. A system for base calling, comprising:
a memory for storing data including a computer-executable program; and
a processor for executing the computer-executable program to implement the method according to any of claims 1 to 26.

28. A sequencing system, comprising the system for base calling according to claim 27.

29. A computer program product comprising an instruction, wherein the steps in the method according to any of claims 1 to 26 are implemented when the program is executed by a computer.

30. A sequencing system, comprising the computer program product according to claim 29.

31. A system for base calling, comprising:
a aligning module configured for aligning the coordinates of spots in a spot set corresponding to a sequencing template to an image to be detected so as to determine a corresponding coordinate position in the image to be detected;
an intensity determination module configured for determining the intensity of the corresponding coordinate position in the image to be detected; and
a calling module configured for comparing the intensity of the corresponding coordinate position in the image to be detected from the intensity determination module with a preset threshold, and performing base calling based on the information of the position in the image to be detected where the intensity is greater than the preset threshold; wherein
the spot set corresponding to the sequencing template is acquired by construction based on a plurality of images; the images and the image to be detected are all acquired from base extensions and correspond to the same field of view;
and a plurality of molecules carrying an optically detectable label are present in the field of view during the base extensions, and at least some of the nucleic acid molecules are present as spots in the images and/or the image to be detected.

32. The system according to claim 31, wherein one preset threshold corresponds to one or more images to be detected.

33. The system according to claim 32, wherein the intensity of the corresponding coordinate position in the image to be detected is relative intensity, and the relative intensity is determined according to absolute intensity of the corresponding coordinate position and background intensity of the block that the corresponding coordinate position is in.

34. The system according to claim 33, wherein in the intensity determination module, determining the background intensity of the block that the corresponding coordinate position is in comprises:
sorting pixels in an x1 × y1 block that the corresponding coordinate position is in according to the pixel values to obtain a distribution curve of the number of the pixels in the x1 × y1 block, where x1 and y1 are both natural numbers, and x1 × y1 is not less than 100; and
determining the background intensity of the block that the corresponding coordinate position is in based on the distribution curve.

35. The system according to claim 34, wherein the sorting is ascending sorting, and the peak pixel value of the distribution curve is used as the background intensity of the block that the corresponding coordinate position is in; the peak pixel value of the distribution curve is estimated according to the formula *I*_{block} = *I*ⱼ₁ + (*I*ⱼ₂ - *I*ⱼ₃) × t1, where *I*ⱼ₁, *I*ⱼ₂ and *I*ⱼ₃ are the pixel values corresponding to the j 1th percentile, j2th percentile and j3th percentile, respectively; j 1, j2 and j3 are all integers less than 50 and not less than 1; j2 > 8 + j3; and t1 is a first correction coefficient and determined by j 1, j2 and j3.

36. The system according to claim 35, wherein j1 is selected from [1, 40], j2 is selected from [6, 40], j3 is selected from [1, 30], and 40 < j1 + (j2 - j3) × t1 < 50.

37. The system according to claim 36, wherein the preset threshold is selected from [0.85, 0.95].

38. The system according to claim 31, wherein one preset threshold corresponds to a corresponding coordinate position in one image to be detected.

39. The system according to claim 38, wherein the intensity of the corresponding coordinate position in the image to be detected is absolute intensity.

40. The system according to claim 39, further comprising a threshold determination module connected to the calling module and configured for determining the preset threshold, wherein the determining the preset threshold comprises:
sorting pixels in an x2 × y2 block that the corresponding coordinate position is in according to the pixel values to obtain a distribution curve of the number of the pixels in the x2 × y2 block, where x2 and y2 are both natural numbers, and x2 × y2 is not less than 100; and
determining the preset threshold based on the distribution curve.

41. The system according to claim 40, wherein the sorting is ascending sorting, and the right trough pixel value of the distribution curve is used as the preset threshold; the right trough pixel value of the distribution curve is estimated according to the formula Threshold = *I*ⱼ₄ + (*I*ⱼ₅ - *I*ⱼ₆) × t2, where
*I*ⱼ₄, *I*ⱼ₅ and *I*ⱼ₆ are the pixel values corresponding to the j4th percentile, j5th percentile and j6th percentile, respectively;
j4, j5 and j6 are all integers less than 50 and not less than 1; j5 > 8 + j6; and t2 is a second correction coefficient and determined by j4, j5 andj6.

42. The system according to claim 41, wherein j4 is selected from [1, 40], j5 is selected from [6, 40], j6 is selected from [1, 30], and 85 < j4 + (j5 - j6) × t2 < 100.

43. The system according to any of claims 31 to 42, wherein the images include a first image, a second image, a third image and a fourth image corresponding to the same field of view during base extensions of four types of bases A, T/U, G and C, respectively; the first image comprises an image M1 and an image M2, the second image comprises an image N1 and an image N2, the third image comprises an image P1 and an image P2, and the fourth image comprises an image Q1 and an image Q2; and sequential and/or simultaneous completion of the base extensions of the four types of bases once is defined as one cycle of sequencing;
the images M1 and M2 separately come from two cycles of sequencing, the images N1 and N2 separately come from two cycles of sequencing, the images P1 and P2 separately come from two cycles of sequencing, and the images Q1 and Q2 separately come from two cycles of sequencing;
the system further comprises a template construction module connected to the aligning module and configured for implementing the following steps to construct a spot set corresponding to the sequencing template based on a plurality of images:
merging spots in the first image, the second image, the third image and the forth image, recording the number of spots in the same position, and removing spots in a position with a number of 1 to obtain the spot set corresponding to the sequencing template.

44. The system according to claim 43, wherein in the template construction module, the merging spots in the first image, the second image, the third image and the forth image comprises:
(a) merging spots in the image N1 in the image M1 to obtain a one-time merging image M1, marking overlap spots in the image M1 as A and marking non-overlap spots as B, wherein a plurality of spots distanced by less than a first predetermined pixel in the one-time merging image M1 are counted as one overlap spot;
(b) replacing the image N1 with the image PI, the image Q1, the image M2, the image N2, the image P2 or the image Q2, replacing the image M1 with the one-time merging image M1, and repeating (a) multiple times until the spots in all of the images are merged to obtain an original spot set; and
(c) removing the spots marked as B in the original spot set to obtain a spot set corresponding to the sequencing template.

45. The system according to claim 43, wherein the images are registered images.

46. The system according to claim 45, further comprising a registration module connected to the template construction module and configured for implementing the following steps to register the images:
performing a first registration for an image to be registered based on a reference image, wherein the reference image and the image to be registered correspond to the same field of view, comprising:
determining a first offset between a predetermined block in the image to be registered and a corresponding predetermined block in the reference image, and moving all spots in the image to be registered based on the first offset to obtain an image to be registered having undergone the first registration; and
performing a second registration for the image to be registered having undergone the first registration based on the reference image, comprising:
merging the image to be registered having undergone the first registration with the reference image to obtain a merging image;
calculating an offset of second overlap spots in a predetermined block in the merging image to determine a second offset, with a plurality of spots distanced by less than a second predetermined pixel in the merging image defined as a second overlap spot; and
moving all spots in the image to be registered having undergone the first registration based on the second offset to register the image to be registered.

47. The system according to claim 46, wherein the registration module comprises a reference image construction unit for implementing the following steps to construct the reference image:
acquiring a fifth image and a sixth image, wherein the fifth image and the sixth image correspond to the same field of view as the image to be registered;
performing a coarse registration for the sixth image based on the fifth image, comprising: determining an offset of the sixth image relative to the fifth image, and moving the sixth image based on the offset to obtain a sixth image having undergone the coarse registration; and
merging the fifth image with the sixth image having undergone the coarse registration to obtain the reference image.

48. The system according to claim 47, wherein in the reference image construction unit, constructing the reference image further comprises: utilizing a seventh image and an eighth image, wherein the fifth image, the sixth image, the seventh image and the eighth image correspond to the same field of view, and the fifth image, the sixth image, the seventh image and the eighth image correspond to fields of view during base extensions of four types of bases A, T/U, G and C, respectively; and constructing the reference image further comprises:
performing a coarse registration for the seventh image based on the fifth image, comprising: determining an offset of the seventh image relative to the fifth image, and moving the seventh image based on the offset to obtain a seventh image having undergone the coarse registration;
performing a coarse registration for the eighth image based on the fifth image, comprising: determining an offset of the eighth image relative to the fifth image, and moving the eighth image based on the offset to obtain an eighth image having undergone the coarse registration; and
merging the fifth image with the sixth image having undergone the coarse registration, the seventh image having undergone the coarse registration and the eighth image having undergone the coarse registration to obtain the reference image.

49. The system according to any of claims 46 to 48, wherein the reference image and the image to be registered are binarized images.

50. The system according to any of claims 46 to 49, wherein two-dimensional discrete Fourier transform is employed to determine the first offset, the offset of the sixth image relative to the fifth image, the offset of the seventh image relative to the fifth image and/or the offset of the eighth image relative to the fifth image.

51. The system according to any of claims 43 to 50, further comprising a spot detection module connected to the aligning module, the template construction module and/or the registration module and configured for implementing the following steps to detect spots in the images:
preprocessing the image to obtain a preprocessed image;
determining a critical value to simplify the preprocessed image so as to obtain a binarized image, comprising:
assigning a first preset value to a pixel value of a pixel in the preprocessed image less than the critical value, and
assigning a second preset value to a pixel value of a pixel in the preprocessed image not less than the critical value;
determining a first spot detection threshold c1 based on the preprocessed image; and
identifying the spots in the image based on the preprocessed image and the binarized image, comprising: determining a pixel matrix fulfilling at least two of the following conditions i)-iii) as a candidate spot:
i) in the preprocessed image, a center pixel of the pixel matrix has the maximum pixel value, the pixel matrix is represented by r1 × r2, both r1 and r2 are odd numbers greater than 1, and the pixel matrix r1 × r2 comprises r1 × r2 pixels;
ii) in the binarized image, the pixel value of the center pixel of the pixel matrix is the second preset value, and the pixel connectivity in the pixel matrix is greater than (2/3) × r1 × r2; and
iii) in the preprocessed image, the pixel value of the center pixel of the pixel matrix is greater than a third preset value, and g1 × g2 is > c1, where g1 is a correlation coefficient of two-dimensional Gaussian distribution in an m1 × m2 area centered on the center pixel of the pixel matrix, g2 is pixels in the m1 × m2 area, both m1 and m2 are odd numbers greater than 1, and the m1 × m2 area comprises m1 × m2 pixels.

52. The system according to claim 51, wherein in the spot detection module, the following steps are further implemented to determine whether the candidate spot is a spot:
determining a second spot detection threshold based on the preprocessed image, and
comparing the pixel value of the candidate spot with the second spot detection threshold, determining a candidate spot with a pixel value not less than the second spot detection threshold as a spot, and taking the pixel value of the pixel indicated by the coordinates of the candidate spot as the pixel value of the candidate spot.

53. The system according to claim 52, wherein in the spot detection module, determining whether the candidate spot is a spot comprises:
dividing the preprocessed image into a set of blocks of a predetermined size;
sorting pixels in the block according to the pixel values to determine the second spot detection threshold corresponding to the block; and
comparing the pixel value of the candidate spot in the block with the second spot detection threshold, and
determining a candidate spot in the block with a pixel value not less than the second spot detection threshold corresponding to the block as a spot.

54. The system according to any of claims 51 to 53, wherein the preprocessing the image comprises:
determining a background of the image using opening operation;
converting the image based on the background using top-hat operation;
applying a Gaussian blur to the converted image; and
sharpening the Gaussian blurred image to obtain the preprocessed image.

55. The system according to any of claims 51 to 54, wherein the determining a critical value to simplify the preprocessed image so as to obtain a binarized image comprises:
determining the critical value based on the background and the preprocessed image; and
comparing a pixel value of a pixel in the preprocessed image with the critical value so as to obtain the binarized image.

56. The system according to any of claims 51 to 55, wherein g2 is a corrected pixel value in the m1 × m2 area, and pixels in the m1 × m2 area are corrected based on the proportion of pixels of the second preset value in the corresponding m1 × m2 area in the binarized image to obtain the corrected pixels in the m1 × m2 area.
